# EUROPEAN PATENT APPLICATION

(11) **EP 1 749 536 A1**
(43) Date of publication of application: **07.02.2007**
(21) Application number: 05380179.1
(22) Date of filing: 03.08.2005
(51) Int. Cl.: A61K 38/36, A61P 7/04

(54) **Activated factor X stimulants as new antihemorrhagic agents for topical use**

(71) Applicant: Thrombotargets Europe, S.L., 08010 Barcelona (ES)
(72) Inventor: Pedreno Egea, Javier, 08010 Barcelona (ES); Caveda Catasús, Luis, 08010 Barcelona (ES)
(74) Representative: ABG Patentes, S.L.

(57) **Abstract**

The activated coagulation Factor X (FXa) stimulating agents may be used in the treatment of hemorrhaging in a subject. Compounds and combinations are described which are particularly useful for the topical treatment of hemorrhaging in a healthy subject or in a subject with hemorrhagic diathesis.

## Description

### FIELD OF THE INVENTION

This invention generally relates to the treatment of hemorrhaging in a subject by means of the use of Tissue factor (TF), a new activated coagulation factor X (FXa) stimulator. More specifically, the invention is related on one hand to the topical treatment of hemorrhaging in a healthy subject or in a subject suffering from hemorrhagic diathesis by means of the administration to said subject of a composition comprising lipidated TF alone or combined with FXa and/or with a negatively charged inorganic surface (NCIS).

### BACKGROUND OF THE INVENTION

Hemostasis is the mechanism by means of which living beings respond to a hemorrhage and involves the participation of two processes that become functional immediately after a lesion and remain active for a long period of time. The first of them is known as primary hemostasis and is characterized by the occurrence of vasoconstriction at the vascular lesion site and platelet aggregate formation. The second one is known as secondary hemostasis, being the phase in which the fibrin clot is formed due to the action of the different coagulation cascade proteolytic enzymes.

Platelet aggregate formation plays a key role in hemostasis in capillaries, being particularly relevant in mucocutaneous hemorrhaging; in contrast, fibrin clot formation is much more important in large vessel hemostasis, being more relevant in internal hemorrhaging (gastrointestinal, cerebral, etc.). The following phases can be distinguished during platelet aggregate formation: (i) platelet adhesion to the sub-endothelium surface exposed by the lesion; (ii) release of the granular content of platelets as a response to their activation; (iii) platelet aggregation with the subsequent sequestering and concentration of more platelets at the lesion site; and (iv) binding of fibrinogen as well as other coagulation proteins to the platelet surface to produce thrombin and form the fibrin clot that will allow the plates to become fused and consolidated, thus stabilizing the hemostatic clot.

Several cofactors and proteolytic enzymes participate in the second phase of the blood coagulation process, all referred to as coagulation factors, and it consists of several phases ending with fibrin formation from fibrinogen hydrolysis due to the action of thrombin. Furthermore the thrombin production enhances the platelet aggregate by increasing the activation and aggregation of more platelets. Thrombin is previously formed by proteolytic hydrolysis of an apoenzyme, prothrombin. This proteolysis is carried out by the activated coagulation factor X (FXa), which binds to the surface of the activated platelets and only in the presence of its cofactor, activated coagulation factor V (FVa), and calcium ions, and is able to hydrolyze prothrombin. Coagulation factor X (FX) activation can occur in two separate pathways, the intrinsic pathway and the extrinsic pathway.

The intrinsic pathway consists of a series of reactions in which each proenzyme is hydrolyzed, yielding its active protease form. In each step, the recently formed proteolytic enzyme will catalyze activation of the following proenzyme to successively yield the active form. Activation of the different coagulation factors involved in the intrinsic pathway takes place, therefore, in the manner of a cascade, therefore a deficiency of any of the proteins of the intrinsic pathway blocks activation of the following step, preventing clot formation and increasing hemorrhagic tendency. Deficiencies of different coagulation factors, for example, coagulation factor VIII (FVIII), coagulation factor IX (FIX) or coagulation factor XI (FXI), cause severe hemorrhagic syndromes, such as hemophilia A, B and C, respectively.

In the blood coagulation extrinsic pathway, the Tissue factor (TF), exposed on adventitia cells at the lesion site, binds to circulating coagulation factor VII/activated coagulation factor VII (FVII/FVIIa) to form the TF::FVIIa complex and, in the presence of calcium, to act as a substrate so that FX activation takes place. The extrinsic pathway is currently considered the most relevant pathway in blood coagulation, and it is accepted that in the event of a hemorrhage produced by a vascular lesion, coagulation is triggered due to extrinsic pathway activation involving the interaction of TF with its ligand, FVII/FVIIa. Another role assigned to the TF::FVIIa complex in coagulation is to act as a substrate so that FX activation takes place due to FVIIa. As a result, basal FXa levels, which initially are insufficient to generate fibrin clot formation, increase. This increase in basal FXa concentrations in the presence of its cofactor, activated factor V (FVa), and of a cellular procoagulant surface, would be able to produce the thrombin required for fibrin clot formation. It is currently accepted that once the platelets are activated, they play a key role in blood coagulation. On one hand, they provide the procoagulant surface rich in anionic phospholipids and on the other hand they expose the FVa and FXa factors stored within them. All this allows correct assembly of the different agents involved in coagulation on the surface of their plasma membranes.

The theory of extrinsic pathway activation is capable of explaining how coagulation begins place through the role that has been attributed to the TF::FVIIa complex. Evidently in a context in which FVIIa is not present, as occurs in a congenital deficiency of this factor, coagulation never takes place since FX is not activated at sufficient levels and, as a result, hemorrhagic manifestations are fatal. Murine models confirm this theory and FVII deficiency is incompatible with life, being accompanied by severe fatal hemorrhaging. However, congenital FVII deficiencies in human beings described until now are not always accompanied by hemorrhaging. Cases of complete FVII deficiency with no clinical symptoms and occurring in healthy individuals with no hemorrhagic complications have been reported. All this suggests that there must be other mechanisms independent of FVII in human beings that are able to begin coagulation.

Current management of hemorrhaging (both external and internal) associated with congenital FVII deficiency consists of the parenteral administration of human plasma (with all the associated problems this implies) or the administration of recombinant human FVIIa (rhFVIIa). This, although shown to be useful, also has a series of drawbacks associated thereto that have made different public authorities, such as the Federal Drug Administration (FDA) or the European Agency for the Evaluation of Medicinal Products (EMEA), greatly limit its use. Parenteral administration, which always requires repeated doses, and the extremely high price (each treatment costs an average of 6,000 €), are some of these drawbacks.

TF is an integral membrane glycoprotein belonging to the super-family of class II cytokine receptors specifically bonding to FVII/FVIIa and plays a relevant role in the blood coagulation extrinsic pathway. The physiological roles assigned to TF are well known; on one hand, it is a receptor specific for FVIIa and, once the TF::FVIIa complex has been formed, it acts as a substrate so that FX activation takes place. In fact, after a vascular lesion, TF, which is normally sequestered on the surface of adventitia cells externally surrounding blood vessels, comes into contact and interacts with its ligand, FVII present in blood, to form the TF::FVII complex. Once this complex is formed, FVII autoactivation takes place, yielding its active form (FVIIa). There is currently extensive information on the TF::FVII complex structure. The main FVII binding sites participating in the interaction with TF are located in the first domain similar to that of the epidermal growth factor (EGF) and in the protease domain. On the other hand, it has also been reported that other less relevant binding sites participate (4-carboxyglutamate-rich domain (Gla domain) and the second EGF domain). The binding sites present in TF are located in the two type III fibronectin domains and in the intermediate region between both domains.

The role assigned to the TF::FVIIa complex in coagulation is widely known. The TF::FVIIa complex acts as a substrate so that FX activation takes place. Recent studies have allowed identifying that the TF Lysine 165 and Lysine 166 residues interact with the Gla domain of FX, both in the activated and non-activated forms.

One example illustrating the biological relevance of the TF::FVIIa complex in the blood coagulation process is the Disseminated Intravascular Coagulation (DIC) Syndrome. This clinical condition is associated with the intravascular release of TF and can occur in the course of severe clinical conditions (shock, sepsis, cardiac arrest, major trauma, liver disease, major surgery, bums, etc).

However, in contrast with that which occurs with information referring to the TF::FVIIa complex little is known about the interaction of TF with FXa. It has only been described that TF can act as a FXa cofactor for FVIIa activation. That is, the binding of FXa to TF would stimulate FVIIa activation which, in turn, will increase FX activation.

Until now it has been accepted that TF is the main element responsible for the quickness with which coagulation is initiated. For coagulation to begin, it is absolutely necessary for FX to be activated and begin prothrombin hydrolysis. The source of this FXa has mainly been attributed to the interaction of FVIIa with its receptor, TF. Although it has been described that FXa is present in platelet granules and that it may be exposed on the surface when its activation takes place, the physiological concentration of FXa (< 150 pM) present in blood is insufficient to begin thrombin formation, even in the presence of its cofactor, FVa and of a platelet procoagulant surface. Therefore it is currently accepted that it is necessary for the FXa basal concentration to increase and reach levels causing coagulation to begin. The source of the increase of FXa basal concentrations has always been attributed to the TF::FVIIa complex. On the other hand, it is accepted today that platelet aggregation plays a key role in primary hemostasis, being considered a fundamental element in consolidation of the fibrin clot.

Congenital deficiencies of each coagulation factor can be associated with the occurrence of hemorrhaging. Existing therapies for these deficiencies are based on the administration of plasmas or of the human recombinants in question. Congenital coagulation diseases generally involve a single protein; thus, for example, hemophilia A is a hereditary hemorrhagic disease affecting FVIII, and bleeding episode therapy is fundamentally based on the transfusion of plasmas containing FVIII or recombinant FVIII (rFVIII) obtained by genetic recombination techniques. The von Willebrand disease is another hemorrhagic disease characterized by an extended bleeding time associated with a von Willebrand protein deficiency or abnormality. Treatment of this disease is based on the transfusion of normal plasma or von Willebrand protein-enriched plasma.

Acquired coagulation diseases occur in individuals with no prior history of bleeding and may have multiple sources; by way of illustration, the presence of inhibitors specific for coagulation factors may occur in individuals who have been subjected to many transfusions. Although acquired coagulation factor deficiencies are an unknown etiological entity also causing severe hemostatic problems, they are also one of the most important problems in multiple transfusions to which patients with congenital coagulopathies are subjected.

The currently available therapeutic arsenal in a mild/moderate or severe/fatal mucocutaneous hemorrhage (due to an external trauma) is very limited. There is evidence that different TF recombinants have been able to accelerate coagulation in healthy and hemophilic individual samples, attributing this action to the classic role assigned to TF as an FVIIa receptor. However, its use as a hemostatic agent for topical use both in normal hemostatic conditions and in deficiencies of any coagulation factor has not been described. European patent EP 266993 discloses the use of non-lipidated TF as a hemostatic agent for parenteral treatment of hemorrhagic syndromes.

There are different organic hemostatic agents, such as fibrillar collagen, and inorganic hemostatic agents, such as silica surfaces (and others) that are able to accelerate blood coagulation and preventing hemorrhaging. However neither the formulations nor the compounds on their own allow them to be drugs for topical, and accordingly a very ample use, the reason for which they currently have a very restricted use.

Surprisingly there are no drugs available today useful for the topical treatment of a simple episode of epistaxis (nose bleed) or gingival bleeding after brushing one's teeth or simply due to an everyday wound caused by shaving, due to the punctured vein in a blood extraction, or due to the wound from an accidental fall in the street. The problem is further aggravated in the case of patients with hemorrhagic diathesis, for example with congenital coagulopathies of the hemophilia type or the von Willebrand disease or patients with congenital platelet disorders, of the Glanzmann's disease type or the Bernard-Soulier syndrome, or acquired coagulopathies. These patients have serious problems with day to day living and in a simple dental extraction or in any minor trauma causing a bleeding wound they have no medical treatment available to improve their quality of life. The problem obviously becomes greater when these patients suffer an external trauma or severe bleeding accident since their life is at serious risk. In all these situations, the only available pharmacological tool is the administration of human plasma containing the deficient factors or the human recombinant factor specific for each coagulation factor. All these therapies imply using the parenteral route and, therefore, are not designed to be used with great frequency, as would be the case, for example, in any daily mild or moderate bleeding.

In conclusion, being able to have new pharmacological tools effective in topical treatment of hemorrhagic syndromes whether they are mild/moderate or severe is vitally important, first to increase the quality of life of hemostatically normal individuals and of patients with congenital coagulopathies and/or platelet disorders, and secondly to be able to save the life of any individual with a fatal bleeding wound.

### SUMMARY OF THE INVENTION

The invention object of this patent application is based on the discovery that the lipidated Tissue Factor (TF) has a new role in coagulation. The findings herein described show for the first time that lipidated TF in absence of its ligand FVII/FVIIa acts as an FXa cofactor, allowing this serine protease to begin prothrombin hydrolysis and, accordingly, for coagulation to begin. The inventors' data suggests that the quickness with which coagulation takes place is dependent on the formation of a new complex not disclosed until now, the TF::FXa complex.

In the presence of FXa physiological concentrations (<150 pM) incapable of initiating coagulation on their own, lipidated TF is quickly able to initiate thrombin formation as a result of its action as a cofactor. On the other hand, since it interacts with the FXa exposed on the platelet surface, lipidated TF promotes platelet aggregation mediated by FXa, which leads to the quick formation of the platelet clot and, accordingly, the acceleration of hemostasis.

These hemostatic effects are surprisingly independent of the presence of FVII and FVIIa, therefore the procoagulant effect mediated by the endogenous TF::FXa complex (FXa < 150 pM) is particularly useful and relevant in factor VII-deficient samples. Nevertheless, potent hemostatic effects have also been observed in samples from patients with congenital defects of other coagulation factors, such as: FVIII (hemophilia A), FIX (hemophilia B), FXI (hemophilia C), FV, FX and FXII, as well as in individuals with congenital platelet disorders, such as the Bernard Soulier Syndrome and Glanzmann's Disease.

Therefore, this invention is based on the use of lipidated TF alone or combined with FXa and/or with negatively charged inorganic surfaces (NCIS) as a new FXa proteolytic activity stimulating agent, useful for the topical treatment of hemorrhaging present in healthy individuals and in patients with hemorrhagic diathesis.

The results obtained by the inventors open the doors to the use of non-lipidated Tissue Factor (TF) or a functional fragment thereof for the treatment of hemorrhaging in a coagulation factor VII-deficient subject. Said treatment can be carried out by means of the use of suitable pharmaceutical administration forms for the parenteral administration of non-lipidated TF.

Therefore in one aspect the invention relates to the use of lipidated Tissue Factor (TF), or a functional fragment thereof, in the preparation of a drug for the topical treatment of hemorrhaging in a subject.

In another aspect, the invention relates to a product comprising lipidated TF alone or combined with activated coagulation Factor X (FXa) and/or a negatively charged inorganic surface (NCIS). The use of said product as a drug or in the preparation of a drug for the treatment of hemorrhaging in a subject constitutes a further aspect of this invention.

In another aspect the invention relates to a complex formed by lipidated TF and a compound selected from FXa, an NCIS and combinations of both. The use of said complex as a drug or in the elaboration of a drug for the treatment of hemorrhaging in a subject constitutes a further aspect of this invention.

In another aspect the invention relates to a pharmaceutical composition comprising a lipidated Tissue Factor (TF), together with a pharmaceutically acceptable carrier. In a particular embodiment, said pharmaceutical composition further comprises FXa and/or an NCIS.

In another aspect the invention relates to a product comprising said pharmaceutical composition and a support. In a particular embodiment, said pharmaceutical composition further comprises FXa and/or an NCIS.

In another aspect the invention relates to the use of non-lipidated Tissue Factor (TF), or a functional fragment thereof, for the preparation of a drug for the treatment of hemorrhaging in a coagulation Factor VII-deficient subject. In a particular embodiment said drug is formulated in a pharmaceutical administration form suitable for the parenteral administration of non-lipidated TF.

### DETAILED DESCRIPTION OF THE INVENTION

The findings herein described show for the first time that lipidated TF in the absence of its ligand, FVII/FVIIa, acts as an FXa cofactor causing this enzyme to initiate prothrombin hydrolysis and, accordingly, for coagulation to take place. This new mechanism is particularly relevant in very low FXa concentration levels (below those considered physiological 150 pM). On the other hand, when lipidated TF binds to the FXa exposed on the platelet surface, it induces platelet aggregation mediated by FXa, leading to platelet clot formation and to hemostasis enhancement. Until now TF has not been assigned any role in platelet aggregation; nevertheless the inventors' results show for the first time that lipidated TF is able to aggregate platelets by means of a mechanism involving the participation of intraplatelet FXa and its membrane receptor, EPR-1. The consequence is two-fold; on one hand it causes platelet aggregation and, therefore, the recruitment of more platelets and, on the other hand, it amplifies thrombin formation as a result of the assembly of the prothrombin complex on the platelet surface by means of the exposure of intraplatelet FVa and of the procoagulant surface rich in anionic phospholipids. It is well known that there are certain platelet diseases occurring with disorders in platelet aggregation and a greater tendency of hemorrhagic episodes, amongst which Glanzmann's disease and the Bernard-Soulier Syndrome stand out, in which congenital defects affecting the fibrinogen receptor or the Gplb receptor, respectively, have been disclosed.

Accordingly, exogenously administered lipidated TF is not only useful in congenital coagulopathies but will further allow hemostasis in congenital platelet disorders. The quickness with which coagulation takes place is therefore not dependent on the formation of the TF::FVIIa complex and subsequent FX activation, as has been thought until now, but on the formation of a new complex formed by TF and FXa physiological basal concentrations (<150 pM). The most relevant biological consequence of the formation of this new complex is the quick generation of the beginning of coagulation and that this only requires the presence of FXa physiological concentrations present in blood and the interaction of the enzyme with its cofactor, TF. The process is immediately amplified as a result of the thrombin initially formed by the complex and as a result of the greater production of FXa, whether due to the TF::FVIIa complex or due to thrombin itself.

Therefore the inventors' findings clearly show that in the absence of FVIIa and in the presence of FXa physiological concentrations (< 150 pM) incapable of initiating coagulation on their own, lipidated TF quickly initiates thrombin formation as a result of its new action as an FXa cofactor, promoting the beginning of prothrombin hydrolysis. On the other hand, the inventors' findings show that lipidated TF and FXa at low concentrations (which are not effective for exercising antihemorrhagic effects on their own) when administered together are able to quickly cause the beginning of the fibrin clot formation.

The effects observed with the TF::FXa combinations, specifically with an FXa concentration <150 pM, are much better than those detected when lipidated TF is administered alone or combined with negatively charged inorganic surfaces (NCIS). The obtained results support the idea that in the presence of FXa basal concentrations present in plasma (<150 pM), incapable of generating the initial procoagulant response on their own, this response takes place when TF acts as an FXa cofactor.

Although the combination with an NCIS enhances and amplifies the hemostatic effect of exogenously administered TF, it was always less than that obtained with the administration of lipidated TF and FXa, regardless of whether or not the FXa concentration was less than 150 pM.

Accordingly, lipidated TF, alone or combined with FXa and/or NCIS, can be used as new topical hemostatic agents useful for the treatment of hemorrhaging present in healthy individuals and in patients suffering from hemorrhagic diathesis.

Non-lipidated TF can likewise be used as a new hemostatic agent useful for treatment, preferably by parenteral administration, of hemorrhaging present in FVII-deficient patients.

### I. Use of Lipidated TF in the Topical Treatment of Hemorrhaging

The inventors have surprisingly found that lipidated TF increases FXa proteolytic activity and, accordingly, thrombin production [see section A of the results of the example included in this description] and that it is able to induce platelet aggregation mediated by the interaction of platelet-transported and exposed FXa [see section B of the results of the example included in this description]. The inventors have also observed that lipidated TF coagulates plasma and blood in healthy subjects and coagulation factor-deficient patients, including those that are FVII-deficient, both *in vitro* [see, for example, sections C and E of the results of the example included in this description] and *in vivo* [see sections I and J of the results of the example included in this description]. The inventors have further found that lipidated TF coagulates blood in patients suffering from platelet disorders [see section D of the results of the example included in this description]. These results clearly show that lipidated TF is an antihemorrhagic agent useful for topical treatment of hemorrhaging in a subject.

Therefore in one aspect the invention is aimed at the use of lipidated Tissue Factor (TF), or a functional lipidated fragment thereof, in the preparation of a drug for the topical treatment of hemorrhaging in a subject.

Tissue factor (TF) is an integral membrane glycoprotein that is widely distributed in the animal kingdom which, as it is found naturally, consists of a proteinaceous component (protein) and a phospholipid. The TF protein has a domain structure, i.e. it is a protein with independent functional regions. Each one of the domains of the human TF apoprotein has unique structural and functional characteristics: (1) a signal peptide or a region with a 32 amino acid leader sequence that is post-translationally processed when the protein is processed from the immature to the mature form; (2) an N-glycosylated hydrophilic extracellular domain comprising about 219 terminal amino acids; (3) a fragment of about 23 amino acids, mainly hydrophobic, which are believed to be the transmembrane domain amino acids; and (4) the 21-amino acid carboxyl end which are believed to be the amino acids forming part of the protein cytoplasmic fragment. This domain structure of the human TF protein allows the production of, for example, the extracellular domain of the protein or functional fragments thereof. The amino acid sequence of the human TF protein is known and may be consulted in protein data bases such as, for example, NCBI (human TF, Access number: P13726).

The term "lipidated Tissue Factor (TF)" as used herein refers to the purified and (re)-lipidated TF proteinaceous component, i.e. what the lipid component (phospholipids) has been added to after its purification and which can be prepared, by way of an illustrative, non-limiting example, according to the protocol previously described by Morrisey [see the example included in this description]. In said protocol, non-lipidated TF is incorporated within phospholipid vesicles using a non-ionic detergent, such as N-octyl-beta-D-glucopyranoside, for example. The phospholipids that can be used in lipidated TF according to the invention may have any origin (animal, plant or synthetic). Virtually any phospholipid can be used in the preparation of the lipidated TF of this invention. Illustrative, non-limiting examples of phospholipids that can be used in the preparation of lipidated TF include phosphatidylcholine, phosphatidylserine, phosphatidylethanolamine, etc. The TF proteinaceous component:phospholipid ratio (molar, weight or volumetric) may vary within a wide range, for example, from about 1:50000 to about 1:3000.

The term TF as used herein includes several wild-type TF variants and mutants maintaining at least one of the functions of the wild-type TF, advantageously, at least one of the functions of the wild-type TF relating to coagulation.

In a particular embodiment the lipidated TF used for putting the invention into practice is lipidated human TF and consists of the proteinaceous component of a TF isolated from human tissue inserted in a phospholipid bilayer in a suitable TF proteinaceous component:phospholipid ratio, for example, in a molar ratio of about 1:8700. The isolation and purification of TF can be carried out from several tissues such as cerebral, placental and lung tissue, tissue from different animals such as sheep, cows, rabbits, dogs, human beings, etc.

The term "functional fragment of lipidated TF" as it is used in this description includes although is not limited to peptide derivatives of TF, particularly of the proteinaceous component of TF, including mutants and variants of the proteinaceous component of wild-type TF, which maintain one or more TF functions, preferably functions relating to coagulation, for example the capability of binding to the activated coagulation Factor X (FXa) and/or to negatively charged inorganic surfaces (NCIS) and of developing their antihemorrhagic and vessel forming function during wound healing. The amino acid sequence of said functional fragment of TF can be identical to that of a fragment of the proteinaceous component of wild-type TF or may have insertions, deletions or modifications of one or more amino acids, with the condition that at least one of the functions of wild-type TF are preserved, advantageously at least one function related to coagulation. For the sake of simplicity, the term "lipidated TF" as it is used herein includes any functional fragment of lipidated TF.

The proteinaceous component of the TF used in carrying out this invention may further be part of a fusion protein. In this sense, said fusion protein may contain a region A, consisting of the TF protein or a functional fragment of said TF protein, bound to a region B consisting of another fragment of TF. Said region B is bound to the amino-terminus region of said TF protein or of said fragment of the TF protein, or alternatively said region B may be bound to the carboxyl-terminus region of said TF protein or of said fragment of the TF protein. Both regions A and B may be directly bound or bound through a linker polypeptide between said regions A and B. The fusion protein may be obtained either by chemical synthesis or by means of gene expression of the nucleotide sequence encoding for said fusion protein in suitable host cells.

The term "topical treatment" as used herein refers to the application of the treatment directly at the site where it is required, for example, in discontinuous sections of skin (cuts, etc.) and vascular tissue (ruptured vessels, etc.).

According to this invention and as shown in the example included in this description, lipidated TF acts as an FXa proteolytic activity stimulating agent (cofactor), increasing its proteolytic activity, and accordingly prothrombin production, and it can therefore be used to treat or correct hemorrhagic disorders, particularly those hemorrhagic disorders associated with hemorrhagic diathesis.

The term "hemorrhagic diathesis" refers to the process causing a hemostatic disorder and which as a result gives rise to the occurrence of a hemorrhagic syndrome which may occasionally occur with extended and excessive bleeding. Hemorrhagic diathesis may be caused by a coagulopathy and/or by a platelet disorder or dysfunction.

The term "coagulopathy" refers to a coagulation factor disorder. This disorder may be due to a specific coagulation factor deficiency or deficit, the consequence of which will be the occurrence of a hemorrhagic syndrome, or due to a coagulation factor disorder. The coagulopathy may generally be a congenital coagulopathy or an acquired coagulopathy.

As illustrative, non-limiting examples of congenital coagulopathies, deficiencies of coagulation factors selected from coagulation Factor V (FV), coagulation Factor VII (FVII), coagulation Factor VIII (FVIII), the deficit or deficiency of which causes hemophilia A, coagulation Factor IX (FIX) the deficit or deficiency of which causes hemophilia B, coagulation Factor X (FX), coagulation Factor XII (FXII), coagulation Factor XIII (FXIII) and their combinations, can be mentioned.

Acquired coagulopathies may have different origins. Illustrative examples include coagulation factor synthesis deficiencies in severe hepatic failure, anticoagulant overdose, heparin (which stimulates antithrombin 3 and predominantly inhibits the intrinsic pathway of coagulation) and oral anticoagulants or dicumarins (which are anti-vitamin K and mainly inhibit the extrinsic pathway). An alternative mechanism is based on an exaggerated consumption of coagulation factors such that they are not available to form the clot in a bleeding lesion. This mechanism occurs in the disseminated intravascular coagulation syndrome or coagulopathy due to consumption occurring in multiple illnesses such as in severe sepsis damaging the microcirculation endothelium activating platelets and coagulation factors with the formation of multiple microthrombi; in blood invasion by TF such as placental release; in the retention of a dead fetus; in multiple traumas with the crushing of tissues; in poisonous snake bites, etc. In vasculitis, parietal and endothelial damage releases coagulation activators. The consumption of coagulation factors is worsened by lysis of the fibrin of numerous microthrombi due to the action of plasmin with PDF release, which are antiplatelets and anticoagulants.

The term "platelet disorder" refers to a disorder both in the number and in functional ability of platelets, the result of which is the occurrence of a hemorrhagic syndrome. Said platelet disorder may be congenital or acquired.

In a particular embodiment, said platelet disorder is a congenital platelet disorder. Illustrative, non-limiting examples of congenital platelet disorders include Glanzmann's disease, Bernard Soulier disease, Bolin-Jamieson syndrome, Wiskott-Aldrich syndrome, Paris-Trousseau-Jacobsen syndrome, X chromosome thrombocytopenia, Gray platelet syndrome, Sebastian syndrome and Fanconi anemia.

In another particular embodiment said platelet disorder is an acquired platelet disorder. Illustrative, non-limiting examples of acquired platelet disorders include myeloproliferative disorders, such as thrombocythemia, polycythemia, chronic myelocytic leukemia, etc.; there are functional platelet disorders in myeloid metaplasia with increased bleeding time, glass bead retention defects, platelet aggregation defect, abnormal release, and platelet factor III defect. Functional platelet defects have been found in dysproteinemias in scurvy and in congenital heart disease and cirrhosis.

The term "subject" as used herein includes any member of an animal species, including the human species; by way of an illustrative, non-limiting example, said subject can be a mammal, such as a primate, a domestic animal, a rodent, etc., said subject is preferably a man or woman of any age and race. In a particular embodiment said subject is a human being with no history of hemostasis disorders, such as an individual having no coagulopathies or platelet disorders. In another particular embodiment said subject is a human being having a history of hemostasis disorders, such as an individual having hemorrhagic diathesis, for example, a coagulopathy, such as a congenital or acquired coagulopathy, or a platelet disorder, such as a congenital or acquired platelet disorder.

Therefore in a particular embodiment, the invention relates to the use of lipidated TF in the preparation of a drug for the topical treatment of hemorrhaging in a human being with no history of hemostasis disorders. In another particular embodiment the invention relates to the use of lipidated TF in the preparation of a drug for the topical treatment of hemorrhaging in a human being having a hemorrhagic diathesis.

For administration to the subject, the lipidated TF will be formulated in a pharmaceutical form suitable for its topical administration for topical (local) treatment of hemorrhaging. Illustrative, non-limiting examples of said pharmaceutical forms include aerosols, solutions, suspensions, emulsions, gels, salves, creams, dressings, patches, ointments, mouthwashes, etc. To that end the pharmaceutical formulation comprising lipidated TF will include the pharmaceutically acceptable carriers and excipients required for preparing the chosen pharmaceutical administration form (for more information see the section relating to "Pharmaceutical Composition" in this description).

The lipidated TF dose to be administered to the subject may vary within a very broad range, for example, between about 0.01 µg protein/ml and 100 µg of protein/ml. The lipidated TF dose to be administered will depend on several factors, including among them the features of the TF protein used, such as for example, its activity and biological half life, concentration of the TF protein in the formulation, the clinical condition of the subject or patient, the hemorrhagic disorder to be treated, etc. (for more information see the section relating to "Pharmaceutical Composition" in this description).

### II. Combination Products and Applications

### II.1 lipidated TF + FXa

As is known, in the extrinsic pathway of blood coagulation TF binds to circulating FVII/FVIIa to form the TF::FVII complex and, in the presence of calcium, to act as a substrate so that FX activation takes place. Activation of the extrinsic pathway involves the interaction of TF with its ligand, FVII/FVIIa. This complex, TF::FVIIa, acts as a substrate so that FX activation by FVIIa takes place. Now the inventors have surprisingly found that lipidated TF together with FXa, even in FVII-deficient subjects, induces coagulation, such that lipidated TF acts as an FXa stimulator, allowing this serine protease to initiate prothrombin hydrolysis, to produce prothrombin and for the origin of coagulation to take place. As used herein, "activated coagulation Factor X" or "FXa", refers to a protein, particularly a serine protease, which in its active form is responsible for initiating prothrombin hydrolysis and accordingly gives rise to the beginning of coagulation. This proteolysis is performed by FXa binding to the activated platelet surface and, in the presence of FVa and ionic calcium, hydrolyzing prothrombin.

More specifically, the inventors have surprisingly found that the combination of lipidated TF and FXa, even at such low FXa concentrations that they are incapable of inducing coagulation on their own, coagulates plasma and blood in healthy subjects and in coagulation factor-deficient patients, both *in vitro* [see, for example, sections D and E of the results of the example included in this description] and *in vivo* [see sections I and J of the results of the example included in this description].

Therefore in another aspect the invention relates to a product comprising (i) lipidated Tissue Factor (TF) and (ii) activated coagulation Factor X (FXa). Based on the previously mentioned results, said product can be used as a drug, and it can particularly be used in the treatment of hemorrhaging, for example, in the topical treatment of hemorrhaging, in a subject. Said components (i) and (ii) can be together or separate. In a particular embodiment, the lipidated TF used in the preparation of this product is lipidated human TF.

In a particular embodiment, the invention provides a product comprising (i) lipidated Tissue Factor (TF) and (ii) activated coagulation Factor X (FXa). Both components can be combined and be together in the same composition before their administration to the subject.

In another particular embodiment the invention provides a product comprising, separately, (i) lipidated TF and (ii) FXa. In another particular embodiment the invention provides a product comprising, separately, (i) lipidated TF and (ii) FXa, as a combination for their simultaneous or successive administration to a subject. The combined administration of said components (i) and (ii) to the subject can be carried out simultaneously or sequentially, spaced out in time, in any order, i.e. first lipidated TF and then FXa can be administered, or vice versa. Alternatively, said lipidated TF and FXa can be simultaneously administered.

For its administration to a subject, the previously defined product will be formulated in a pharmaceutical administration form, preferably a pharmaceutical administration form suitable for its topical administration, to which end the pharmaceutically acceptable carriers and excipients suitable for the preparation of the desired pharmaceutical administration form will be incorporated. Information about said carriers and excipients, as well as about said administration forms suitable for the administration of said product of the invention, can be found in galenic pharmacy treatises. For more information see the section relating to the "Pharmaceutical Composition" of this description.

The lipidated TF and FXa dose to be administered to the subject may vary within a wide range. By way of illustration, the lipidated TF dose to be administered may be comprised between about 0.01 µg of protein/ml and 100 µg of protein/ml. Also, by way of illustration, the FXa dose to be administered may be comprised between about 0.001 µg of protein and 10 µg of protein/ml. Generally, the lipidated TF and FXa doses to be administered will depend on several factors, including the characteristics of the TF protein used and the FXa, such as for example, their activity and biological half life, the concentration of the FXa and TF protein in the formulation, the clinical condition of the subject or patient, the hemorrhagic disorder to be treated, etc. (for more information see the section relating to the "Pharmaceutical Composition" of this description).

The weight ratio of the lipidated TF and FXa present in said product may vary within a wide range; by way of illustration, said TF:FXa weight ratio is comprised between 10⁶:1 and 10:1, although other weight ratios are also possible; in a particular embodiment said product of the invention comprises lipidated TF and FXa at a weight ratio of 1 to 0.001, i.e. for each milligram of TF protein there is 1 microgram FXa protein present in the mixture.

The use of the product previously defined as a drug, specifically the use of said product in the preparation of a drug for the treatment of hemorrhaging in a subject, particularly for the topical treatment of hemorrhaging in a subject, constitute further aspects of this invention.

### II.2 Lipidated TF + NCIS

The inventors have also surprisingly found that the combination of lipidated TF and negatively charged inorganic surfaces (NCIS) coagulates plasma and blood of healthy subjects and of coagulation factor-deficient patients both *in vitro* [see section G of the results of the example included in this description] and *in vivo* [see sections I and J of the results of the example included in this description].

The term "negatively charged inorganic surface" or "NCIS" as used in this description includes, though is not limited to, exogenous surfaces substituting endogenous surfaces, which are sites where the assembly of the different coagulation components takes place, such as platelet plasma membranes. There are commercially available NCIS such as, for example, those marketed with the Dade® Actin® (Dade Behring) trademark, e.g., Dade® Actin® FS. By way of an illustrative, non-limiting example, in a particular embodiment a solid support formed by negatively charged anionic phospholipids, such as phosphatidylserine, and accelerators, such as for example ellagic acid, kaolin, celite or micronized silica, and which simulates platelet surfaces, has been used. Different inorganic surfaces able to increase procoagulant activity of TF have been disclosed in the state of the art, see for example US patent application US2003/0133990 Al (Hursey *et al.*), the teachings of which are herein incorporated by reference.

Therefore in another aspect the invention relates to a product comprising (i) lipidated Tissue Factor (TF) and (ii) a negatively charged inorganic surface (NCIS). Based on the previously mentioned results, said product can be used as a drug, and it can particularly be used in treatment of hemorrhaging, for example, in the topical treatment of hemorrhaging, in a subject. Said components (i) and (ii) can be together or separate.

In a particular embodiment the invention provides a product comprising (i) lipidated Tissue Factor (TF) and (ii) a negatively charged inorganic surface (NCIS). Both components can be combined and be together in the same composition before their administration to the subject.

In another particular embodiment the invention provides a product comprising, separately, (i) lipidated TF and (ii) NCIS. In another particular embodiment the invention provides a product comprising, separately, (i) lipidated TF and (ii) NCIS, as a combination for their simultaneous or successive administration to a subject. The combined administration of said components (i) and (ii) to the subject can be carried out simultaneously or sequentially, spaced out in time, in any order, i.e. first the lipidated TF and then the NCIS can be administered, or vice versa. Alternatively, said lipidated TF and NCIS can be simultaneously administered.

For its administration to a subject, the previously defined product will be formulated in a pharmaceutical administration form, preferably a pharmaceutical administration form suitable for its topical administration, to which end the pharmaceutically acceptable carriers and excipients suitable for the preparation of the desired pharmaceutical administration form will be incorporated. Information about said carriers and excipients, as well as about said administration forms suitable for the administration of said product of the invention, can be found in galenic pharmacy treatises. For more information see the section relating to the "Pharmaceutical Composition" of this description.

The lipidated TF and NCIS dose to be administered to the subject may vary within a wide range. By way of illustration, the lipidated TF dose to be administered may be comprised between about 0.01 µg of protein/ml and 100 µg of protein/ml. Also, by way of illustration, the NCIS dose (by volume) to be administered may be comprised between about 10 and 100 µl for each µg of lipidated TF protein. Generally, the lipidated TF and NCIS doses to be administered will depend on several factors, including the characteristics of the TF protein used and the NCIS, such as for example, their activity and biological half life, the concentration of the FXa and NCIS protein in the formulation, the clinical condition of the subject or patient, the hemorrhagic disorder to be treated, etc. (for more information see the section relating to the "Pharmaceutical Composition" of this description).

The weight/volume (w/v) ratio of lipidated TF and NCIS present in said product may vary within a wide range; by way of illustration said lipidated TF:NCIS w/v ratio is comprised between 1:1 and 1:10⁶, although other w/v ratios are also possible; in a particular embodiment, said product of the invention comprises between 10 and 100 µl of NCIS for each µg of lipidated TF protein.

The use of the product previously defined as a drug, specifically the use of said product in the preparation of a drug for the treatment of hemorrhaging in a subject, particularly for the topical treatment of hemorrhaging in a subject, constitute further aspects of this invention.

### II.3 Lipidated TF + FXa + NCIS

The inventors have also surprisingly found that the combination of lipidated TF, FXa and NCIS coagulates plasma and blood of healthy subjects and of coagulation factor-deficient patients *in vitro* [see section H of the results of the example included in this description].

Therefore in another aspect the invention relates to a product comprising (i) lipidated Tissue Factor (TF), (ii) activated coagulation Factor X (FXa) and (iii) a negatively charged inorganic surface (NCIS). Based on the previously mentioned results, said product can be used as a drug, and it can particularly be used in the treatment of hemorrhaging, for example, in the topical treatment of hemorrhaging, in a subject. Said components (i), (ii) and (iii) can be together or separate. Alternatively, two of the components can be together, for example, (i)+(ii), (i)+(iii) or (ii)+(iii), and the third one separate.

In a particular embodiment the invention provides a product comprising (i) lipidated Tissue Factor (TF), (ii) activated coagulation Factor X (FXa) and (iii) a negatively charged inorganic surface (NCIS). Said components can be combined and be together in the same composition before their administration to the subject.

In another particular embodiment the invention provides a product comprising, separately, (i) lipidated TF, (ii) FXa and (iii) NCIS. In another particular embodiment the invention provides a product comprising, separately, (i) lipidated TF, (ii) FXa and (iii) NCIS, as a combination for their simultaneous or successive administration to a subject. The combined administration of said components (i), (ii) and (iii) to the subject can be carried out simultaneously or sequentially, spaced out in time, in any order, i.e. first the lipidated TF, then the FXa, and then the NCIS can be administered, or first the lipidated TF, then the NCIS, and then the FXa can be administered, or first the FXa, then the lipidated TF, and then the NCIS can be administered, or first the FXa, then the NCIS, and then the lipidated TF can be administered, or first the NCIS, then the FXa, and then the lipidated TF can be administered, or first the NCIS, then the lipidated TF and then the FXa can be administered. Alternatively, any two of said components can be mixed in the same composition and be administered together while the third one can be added before or after said binary component composition. In another alternative embodiment said lipidated TF, FXa and NCIS are simultaneously administered.

For its administration to a subject, the previously defined product will be formulated in a pharmaceutical administration form, preferably a pharmaceutical administration form suitable for its topical administration, to which end the pharmaceutically acceptable carriers and excipients suitable for the preparation of the desired pharmaceutical administration form will be incorporated. Information about said carriers and excipients, as well as about said administration forms suitable for the administration of said product of the invention, can be found in galenic pharmacy treatises. For more information see the section relating to the "Pharmaceutical Composition" of this description.

The lipidated TF, FXa and NCIS dose to be administered to the subject may vary within a wide range. By way of illustration, the lipidated TF dose to be administered may be comprised between about 0.01 µg of protein/ml and 100 µg of protein/ml. Also, by way of illustration, the FXa dose to be administered may be comprised between about 0.001 µg of protein and 10 µg of protein/ml. Additionally, by way of illustration, the NCIS dose (by volume) to be administered may be comprised between about 10 and 100 µl for each µg of lipidated TF protein. Generally, the lipidated TF, FXa and NCIS doses to be administered will depend on several factors, including the characteristics of the TF protein, FXa and NCIS used, such as for example, their activity and biological half life, the concentration of the TF protein, FXa and NCIS in the formulation, the clinical condition of the subject or patient, the hemorrhagic disorder to be treated, etc. (for more information see the section relating to the "Pharmaceutical Composition" of this description).

The weight ratio of the lipidated TF, FXa and NCIS present in said product may vary within a wide range, as previously mentioned in sections II.1 and II.2; nevertheless, in a particular embodiment, said product of the invention comprises lipidated TF:FXa:NCIS at a 1:0.001:100 (w:w:v) ratio.

The use of the product previously defined as a drug, specifically the use of said product in the preparation of a drug for the treatment of hemorrhaging in a subject, particularly for the topical treatment of hemorrhaging in a subject, constitute further aspects of this invention.

### III. Complexes and Applications

### III.1 TF::FXa complex

As previously mentioned (see section II.1 of this description), the inventors have found that the combination of lipidated TF and FXa, even at such low FXa concentrations that they are incapable of inducing coagulation on their own, coagulates plasma and blood in healthy subjects and in coagulation factor-deficient patients both *in vitro* [see, for example, sections D and E of the results of the example included in this description] and *in vivo* [see sections I and J of the results of the example included in this description]. Though it is not intended to be linked to any theory, it is thought that the administration, either combined or separate (in any order), of said lipidated TF and FXa gives rise to the formation of a complex that is able to exercise the therapeutic effect (antihemorrhagic, particularly, topical antihemorrhagic) observed at the site where said therapeutic effect must be exercised.

Therefore in another aspect, the invention relates to a complex, identified as TF::FXa in this description, comprising lipidated Tissue Factor (TF) and activated coagulation Factor X (FXa). Although for the sake of simplicity said complex is represented as TF::FXa, said complex could actually be formed by several units of each of said components; all these possibilities are within the scope of the invention. In view of the previously mentioned results, said complex can be used as a drug, and it can particularly be used in the treatment of hemorrhaging, for example, in the topical treatment of hemorrhaging, in a subject. In a particular embodiment, the lipidated TF used in the preparation of this complex is lipidated human TF.

For its administration to a subject, the previously defined product will be formulated in a pharmaceutical administration form, preferably a pharmaceutical administration form suitable for its topical administration, to which end the pharmaceutically acceptable carriers and excipients suitable for the preparation of the desired pharmaceutical administration form will be incorporated. Information about said carriers and excipients, as well as about said administration forms suitable for the administration of said product of the invention, can be found in galenic pharmacy treatises. For more information see the section relating to the "Pharmaceutical Composition" of this description.

The lipidated TF and FXa doses present in said complex to be administered to the subject may vary within a wide range. Generally, said doses correspond to the doses previously mentioned in section II.1 relating to a product comprising lipidated TF and FXa.

The weight ratio of the lipidated TF and FXa present in said TF::FXa complex may vary within a wide range; although it generally corresponds to that previously mentioned in II. 1 relating to a product comprising lipidated TF and FXa.

The use of the complex previously defined as a drug, specifically the use of said complex in the preparation of a drug for the treatment of hemorrhaging in a subject, particularly for the topical treatment of hemorrhaging in a subject, constitute further aspects of this invention.

### III.2 TF::NCIS Complex

As previously mentioned (see section II.2 of this description), the inventors have found that the combination of lipidated TF and NCIS coagulates plasma and blood in healthy subjects and in coagulation factor-deficient patients both *in vitro* [see section G of the results of the example included in this description] and *in vivo* [see sections I and J of the results of the example included in this description]. Though it is not intended to be linked to any theory, it is thought that the administration, either combined or separate (in any order), of said lipidated TF and NCIS gives rise to the formation of a complex that is able to exercise the therapeutic effect (antihemorrhagic, particularly, topical antihemorrhagic) observed at the site where said therapeutic effect must be exercised.

Therefore in another aspect, the invention relates to a complex, identified as TF::NCIS in this description, comprising lipidated Tissue Factor (TF) and a negatively charged inorganic surface (NCIS). Although for the sake of simplicity said complex is represented as TF::NCIS, said complex could actually be formed by several units of each of said components; all these possibilities are within the scope of the invention. In view of the previously mentioned results, said complex can be used as a drug, and it can particularly be used in the treatment of hemorrhaging, for example, in the topical treatment of hemorrhaging, in a subject. In a particular embodiment, the lipidated TF used in the preparation of this complex is lipidated human TF.

For its administration to a subject, the previously defined product will be formulated in a pharmaceutical administration form, preferably a pharmaceutical administration form suitable for its topical administration, to which end the pharmaceutically acceptable carriers and excipients suitable for the preparation of the desired pharmaceutical administration form will be incorporated. Information about said carriers and excipients, as well as about said administration forms suitable for the administration of said product of the invention, can be found in galenic pharmacy treatises. For more information see the section relating to the "Pharmaceutical Composition" of this description.

The lipidated TF and NCIS doses present in said complex to be administered to the subject may vary within a wide range. Generally, said doses correspond to the doses previously mentioned in section II.2 relating to a product comprising the combination of lipidated TF and NCIS.

The w/v ratio of the lipidated TF and NCIS present in said TF::NCIS complex may vary within a wide range; although it generally corresponds to that previously mentioned in II.2 relating to a product comprising lipidated TF and NCIS.

The use of the complex previously defined as a drug, specifically the use of said complex in the preparation of a drug for the treatment of hemorrhaging in a subject, particularly for the topical treatment of hemorrhaging in a subject, constitute further aspects of this invention.

### III.3 Lipidated TF::FXa::NCIS Complex

As previously mentioned (see section II.3 of this description), the inventors have found that the combination of lipidated TF, FXa and NCIS coagulates plasma and blood of healthy subjects and of coagulation factor-deficient patients *in vitro* [see section H of the results of the example included in this description]. Though it is not intended to be linked to any theory, it is thought that the administration, either combined or separate (in any order), of said lipidated TF, FXa and NCIS gives rise to the formation of a complex that is able to exercise the therapeutic effect (antihemorrhagic, particularly, topical antihemorrhagic) observed at the site where said therapeutic effect must be exercised.

Therefore in another aspect, the invention relates to a complex, identified as TF::FXa::NCIS in this description, comprising lipidated Tissue factor (TF), activated coagulation Factor X (FXa) and a negatively charged inorganic surface (NCIS). Although for the sake of simplicity said complex is represented as TF::FXa::NCIS, said complex could actually be formed by several units of each of said components, as well as by any possibility of interactions between said components, for example, TF::NCIS::FXa, FXa::NCIS::TF, FXa::TF::NCIS, NCIS::TF::FXa or NCIS::FXa::TF; all these possibilities are within the scope of the invention. In view of the previously mentioned results, said complex can be used as a drug, and it can particularly be used in the treatment of hemorrhaging, for example, in the topical treatment of hemorrhaging, in a subject. In a particular embodiment, the lipidated TF used in the preparation of this complex is lipidated human TF.

For its administration to a subject, the previously defined product will be formulated in a pharmaceutical administration form, preferably a pharmaceutical administration form suitable for its topical administration, to which end the pharmaceutically acceptable carriers and excipients suitable for the preparation of the desired pharmaceutical administration form will be incorporated. Information about said carriers and excipients, as well as about said administration forms suitable for the administration of said product of the invention, can be found in galenic pharmacy treatises. For more information see the section relating to the "Pharmaceutical Composition" of this description.

The lipidated TF, FXa and NCIS present in said complex to be administered to the subject may vary within a wide range. Generally, said doses correspond to the doses previously mentioned in section II.3 relating to a product comprising the combination of lipidated TF, FXa and NCIS.

The ratio of lipidated TF, FXa and the NCIS present in said TF::FXa::NCIS complex may vary within a wide range; although it generally corresponds to that previously mentioned in II.3 relating to a product comprising lipidated TF, FXa and NCIS.

The use of the complex previously defined as a drug, specifically the use of said complex in the preparation of a drug for the treatment of hemorrhaging in a subject, particularly for the topical treatment of hemorrhaging in a subject, constitute further aspects of this invention.

### IV. Pharmaceutical Composition

As previously mentioned, lipidated TF can be used as an antihemorrhagic agent, particularly, as an antihemorrhagic agent for topical application.

Therefore in another aspect, the invention relates to a pharmaceutical composition, hereinafter, pharmaceutical composition of the invention, comprising lipidated Tissue factor (TF) together with a pharmaceutically acceptable carrier. In a particular embodiment, the lipidated TF is lipidated human TF.

For its administration to a subject, the previously defined product will be formulated in a pharmaceutical administration form, preferably a pharmaceutical administration form suitable for its topical administration, to which end the pharmaceutically acceptable carriers and excipients suitable for the preparation of the desired pharmaceutical administration form will be incorporated. Information about said carriers and excipients, as well as about said administration forms suitable for the administration of said product of the invention, can be found in galenic pharmacy treatises. A review of the different pharmaceutical administration forms of drugs in general, and of their preparation processes, can be found in the book "Tratado de Farmacia Galénica" ("Galenic Pharmacy Treatise"), by C. Faulí i Trillo, 1st Edition, 1993, Luzán 5, S.A. of Ediciones.

Although different pharmaceutical administration forms of lipidated TF could be used, administering said compound topically is most advantageous in practice, therefore said lipidated TF will be formulated in a pharmaceutical form suitable for its topical administration. Illustrative, non-limiting examples of said pharmaceutical forms include aerosols, solutions, suspensions, emulsions, gels, salves, creams, dressings, patches, ointments, mouthwashes, etc. To that end the pharmaceutical formulation comprising lipidated TF will include the pharmaceutically acceptable carriers and excipients required for preparing the pharmaceutical administration form of lipidated TF for topical administration.

Therefore in a particular embodiment, the pharmaceutical composition of the invention is a pharmaceutical composition for the topical administration of a lipidated Tissue factor (TF), comprising lipidated TF and a pharmaceutically acceptable carrier suitable for the topical administration of said lipidated TF.

Lipidated TF will be present in the pharmaceutical composition of the invention in a therapeutically effective amount. Said amount may vary within a wide range, for example, between about 0.01 µg of protein/ml and 100 µg of protein/ml.

In another particular embodiment the pharmaceutical composition of the invention comprises:
a) a product comprising (i) lipidated TF and (ii) FXa, together with a pharmaceutically acceptable carrier; or
b) separately, (i) lipidated TF together with a pharmaceutically acceptable carrier, and (ii) FXa together with a pharmaceutically acceptable carrier; or
c) a product comprising (i) lipidated TF and (ii) an NCIS, together with a pharmaceutically acceptable carrier; or
d) separately, (i) lipidated TF together with a pharmaceutically acceptable carrier, and (ii) an NCIS together with a pharmaceutically acceptable carrier; or
e) a product comprising (i) lipidated TF, (ii) FXa and (iii) an NCIS, together with a pharmaceutically acceptable carrier; or
f) separately, (i) lipidated TF together with a pharmaceutically acceptable carrier, (ii) FXa together with a pharmaceutically acceptable carrier, and (iii) an NCIS together with a pharmaceutically acceptable carrier; or
g) a TF::FXa complex together with a pharmaceutically acceptable carrier; or
h) a TF::NCIS complex together with a pharmaceutically acceptable carrier; or
i) a TF::FXa::NCIS complex together with a pharmaceutically acceptable carrier.

The previously defined pharmaceutical composition of the invention contains, as can be seen, lipidated TF alone or combined with FXa and/or NCIS or forming complexes with FXa and/or NCIS, as the active ingredient. In a particular embodiment, the lipidated TF present in the previously defined pharmaceutical composition of the invention is lipidated human TF.

Also in a particular embodiment, the previously defined pharmaceutical composition of the invention will be formulated in a pharmaceutical form for the topical administration of the active ingredient (lipidated TF alone or combined with FXa and/or NCIS or forming complexes with FXa and/or NCIS). Illustrative, non-limiting examples of said pharmaceutical forms include aerosols, solutions, suspensions, emulsions, gels, salves, creams, dressings, patches, ointments, mouthwashes, etc.

To that end, the pharmaceutical formulation comprising the previously mentioned active ingredient will include the pharmaceutically acceptable carriers and excipients required for the preparation of the pharmaceutical administration form of said active ingredient by topical administration.

The active ingredient will be present in the pharmaceutical composition of the invention in a therapeutically effective amount. The active ingredient dose to be administered to a subject will depend, among other factors, on the severity of the pathology said subject suffers from, on the chosen pharmaceutical administration form, etc. For this reason the doses mentioned in this invention must be considered only as guides for a person skilled in the art, and this person must adjust the doses according to the previously mentioned variables. Nevertheless, the pharmaceutical composition of the invention can be administered one or more times a day for preventive or therapeutic purposes.

The pharmaceutical composition of the invention can be used together with other additional drugs useful in the prevention and/or treatment of a hemorrhagic diathesis (e.g., coagulation factors, human plasma, etc.) to provide a combination therapy. Said additional drugs can be part of the same pharmaceutical composition or, alternatively, they can be provided in the form of a separate composition for their simultaneous or successive (sequential in time) administration with respect to the administration of the pharmaceutical composition of the invention.

### V. Supported Pharmaceutical Composition

The pharmaceutical composition of the invention can be placed on a support.

Therefore in another aspect the invention relates to a product comprising the pharmaceutical composition of the invention and a support.

The term "support" as used herein refers to a substrate of suitable material allowing depositing the pharmaceutical composition of the invention thereon, its being carried and its release at the desired site, for example, in the site where the pharmaceutical composition of the invention exercises its therapeutic effect. Said support can be a solid support or a non-solid support, for example, a liquid support or a gaseous support. Illustrative, non-limiting examples of solid supports include dressings, band-aids, compresses, plasters, etc. Illustrative, non-limiting examples of liquid supports include gels, sprays, mouthwashes, etc. Illustrative, non-limiting examples of gaseous supports include air, propellants, etc.

In a particular embodiment the pharmaceutical composition of the invention deposited on said support comprises:
(a) (i) a support, (ii) a product comprising lipidated TF and FXa, together with a pharmaceutically acceptable carrier, (iii) a product comprising lipidated TF and an NCIS, together with a pharmaceutically acceptable carrier and (iv) a product comprising lipidated TF, FXa and an NCIS, together with a pharmaceutically acceptable carrier; or
(b) a TF::FXa complex, together with a pharmaceutically acceptable carrier, or
(c) a TF::NCIS complex, together with a pharmaceutically acceptable carrier, or
(d) a TF::FXa::NCIS complex, together with a pharmaceutically acceptable carrier.

In a particular embodiment the lipidated TF present in the pharmaceutical composition of the invention is lipidated human TF.

This product comprising the pharmaceutical composition of the invention deposited on a support can be obtained by conventional methods, for example, by mixing the pharmaceutical composition of the invention and the support. The interaction between the pharmaceutical composition of the invention and the support can be a physical or chemical interaction, depending on the nature of the components of the pharmaceutical composition of the invention and on the support used.

### VI. Use of Non-lipidated TF for the Treatment of Hemorrhaging due to FVII Deficiency

The results obtained in the assays carried out by the inventors clearly show that TF:
- increases the proteolytic activity of FXa, and accordingly thrombin production, and that it is able to induce platelet aggregation mediated by interaction with the platelet-transported and exposed FXa;
- coagulates the plasma and blood of healthy subjects and of coagulation factor-deficient patients, including FVII-deficient blood, both *in vitro* and *in vivo;* and
- coagulates the blood of patients suffering from platelet disorders.

Therefore these results as a whole clearly show that TF is an antihemorrhagic agent useful for the treatment of hemorrhaging in a subject, including in FVII-deficient patients.

European patent EP 266993 discloses the use of non-lipidated TF in the treatment of hemorrhaging, particularly, in the treatment of hemorrhaging due to a deficiency of a coagulation factor, specifically of a coagulation factor chosen from FVIII, FIX, FXI or FXIII. However, said patent neither discloses nor suggests the possibility of using non-lipidated TF in the treatment of hemorrhaging due to FVII deficiency.

The discovery now made by the inventors relating to the role of TF as an FXa proteolytic activity stimulating agent regardless of whether the subject is FVII-deficient or not, allows establishing the possibility of using non-lipidated TF, or a functional non-lipidated fragment thereof, in the treatment of hemorrhaging in an FVII-deficient subject.

Therefore in another aspect the invention relates to the use of non-lipidated Tissue factor (TF), or a functional non-lipidated fragment thereof, for the preparation of a drug for the treatment of hemorrhaging in an FVII-deficient subject.

The term "non-lipidated Tissue factor (TF)" as used herein refers to the purified, non-lipidated proteinaceous component of TF, i.e. it lacks the lipid component (phospholipid) of TF as it is naturally found. The term TF as used herein includes wild-type TF variants and mutants maintaining at least one of the wild-type TF functions, advantageously at least one of the wild-type TF functions relating to coagulation. The isolation and purification of TF can be carried out from several tissues such as cerebral, placental and lung tissue, tissue from different animals such as sheep, cows, rabbits, dogs, human beings, etc. In a particular embodiment, the non-lipidated TF used for putting the invention into practice is non-lipidated human TF and consists of the proteinaceous component of a TF isolated from human tissue.

The term "functional fragment of non-lipidated TF" as used in this description, includes, although it is not limited to, peptide derivatives of TF, particularly of the proteinaceous component of TF, including mutants and variants of the proteinaceous component of wild-type TF, which maintain one or more TF functions, preferably functions relating to coagulation, for example the capability of binding to the activated coagulation Factor X (FXa) and/or to negatively charged inorganic surfaces (NCIS) and of developing their antihemorrhagic and vessel forming function during wound healing. The amino acid sequence of said functional fragment of TF can be identical to that of a fragment of the proteinaceous component of wild-type TF or may have insertions, deletions or modifications of one or more amino acids, with the condition that at least one of the functions of wild-type TF are preserved, advantageously at least one function relating to coagulation. For the sake of simplicity, the term "non-lipidated TF" as it is used herein includes any functional fragment of non-lipidated TF.

The non-lipidated TF can be obtained by conventional methods. By way of illustration, the proteinaceous component of TF is disassociated from the lipid component by means of extraction with organic solvents. Illustrative examples of said organic solvent include pyridine, ethanol, heptane-butanol mixtures, etc. The proteinaceous component of TF can be purified by means of conventional chemical processes. Examples of said chemical processes include treatment with detergents, for example deoxycholate, Triton X-100, etc., gel filtration and preparative electrophoresis in polyacrylamide gels in the presence of sodium dodecyl sulfate, the use of concavalin A bound to a Sepharose column, the use of affinity columns using antibodies for the proteinaceous component (protein) of TF, etc.

For its administration to an FVII-deficient subject, non-lipidated TF will be formulated in a pharmaceutical form suitable for its administration by any suitable route. Though virtually any pharmaceutical form can be used, the use of pharmaceutical forms for the parenteral administration of said non-lipidated TF is advantageous.

Therefore in a particular embodiment the invention relates to the use of non-lipidated TF, or a non-lipidated fragment thereof, in the preparation of a drug for the parenteral administration of said non-lipidated TF or non-lipidated fragment thereof.

In this case the pharmaceutical compositions containing non-lipidated TF and pharmaceutically acceptable excipients or carriers, will be adapted for their parenteral administration in the form of, for example, sterile solutions, suspensions or lyophilized products in the suitable dosage form; in this case, said pharmaceutical compositions will include suitable excipients, such as buffers, surfactants, etc. In any case, the excipients will be chosen according to the chosen pharmaceutical administration. Information about said carriers and excipients, as well as about said administration forms suitable for the administration of said product of the invention, can be found in Galenic pharmacy treatises. A review of the different pharmaceutical administration forms of drugs in general, and of their preparation processes, can be found in the book "Tratado de Farmacia Galénica" ("Galenic Pharmacy Treatise"), by C. Faulí i Trillo, 1st Edition, 1993, Luzán 5, S.A. of Ediciones.

The dose of non-lipidated TF to be administered to an FVII-deficient subject may vary within a wide range, for example, between about 0.01 µg of protein/ml and 100 µg of protein/ml. The non-lipidated TF dose to be administered will depend on several factors, including the characteristics of the TF protein used, such as, for example, its activity and biological half life, TF protein concentration in the formulation, the clinical condition of the patient, the hemorrhagic disorder to be treated, etc.

The following example illustrates the invention and must not be considered to be limiting thereof.

### EXAMPLE

### TF, alone or combined with FXa and/or negatively charged inorganic surfaces, as an FXa proteolytic activity stimulating agent, useful as an antihemorrhagic agent

For the purpose of evaluating the capacity of TF, alone or combined with FXa and/or negatively charged inorganic surfaces, as an FXa proteolytic activity stimulating agent, a series of *in vitro* and *in vivo* assays were performed, specifically:
- *in vitro* assays demonstrating that in the absence of FVII, TF acts as a factor Xa stimulating agent (chromogenic assays of FXa amidolytic activity, in a solution and in a suspension of washed platelets, and chromogenic assays of the thrombin forming activity in a solution and in a suspension of washed platelets);
- *in vitro* assays demonstrating the coagulant effect of TF in plasma and blood of FVII-deficient patients (coagulation assays in plasma and coagulation assays in non-anticoagulated whole blood);
- *in vitro* assays demonstrating the synergistic effect of TF on FXa used at low concentrations which are incapable of inducing coagulation on their own (coagulation assays in plasma, and (ii) coagulation assays in non-anticoagulated whole blood);
- *in vitro* assays demonstrating the coagulant effect of TF (alone and associated with low FXa concentrations and/or combined with negatively charged inorganic surfaces) in plasma and blood of coagulation factor-deficient patients (coagulation assays in coagulation factor-deficient plasma and coagulation assays in human blood from A and B hemophilic patients);
- *in vitro* assays demonstrating the coagulant effect of TF (alone and combined with negatively charged inorganic surfaces) in blood from patients suffering from platelet disorders (coagulation assays in non-anticoagulated whole blood from patients with platelet disorders);
- *in vivo* assays demonstrating that TF (alone and associated with low FXa concentrations and/or combined with negatively charged inorganic surfaces) is an agent useful for topical antihemorrhagic treatment; and
- *in vivo* assays demonstrating that TF (alone and associated with low FXa concentrations and/or combined with negatively charged inorganic surfaces) is an agent useful for antihemorrhagic treatment by applying directly on the blood vessel.

### I. MATERIALS AND METHODS

### Materials

Recombinant Tissue factor (rTF) was used as a source of lipidated Tissue Factor (TF), specifically the commercial preparation Human Recombinant Tissue Factor (Non Lipidated) (American Diagnostica, USA), relipidated following the method described by Morrissey. For simplicity, in this example, the term rTF or TF refers to lipidated TF unless otherwise stated.

Haematologic Technologies commercial compounds were used as sources of FXa, FII (prothrombin) and FVa.

Commercial Dade® Actin® FS reagent (Dade Behring) was used as negatively charged inorganic surfaces (NCIS).

### Methods

### Method for Relipidating TF in Phospholipid Vesicles Using Dialysis with Octylglucoside (Morrisey Method)

Non lipidated TF is incorporated into phospholipid vesicles using non ionic detergent N-octyl-beta-D-glucopyranoside (octylglucoside). Both TF and the phospholipids are dissolved in octylglucoside forming micelles. Octylglucoside can be easily removed from the solution by dialysis due to its high critical micelle concentration (CMC = 20 to 50 mM). When the octylglucoside is removed the phospholipids are organized in unilamellar vesicles. TF is soaked in these vesicles by virtue of its transmembrane domain. Normally, 50 to 80% of TF molecules are arranged facing outwards from the vesicles.

### Buffers and stock solutions

### Octylglucoside (n-octyl-beta-D-glucopyranoside) from Calbiochem.

Phospholipids:

| | **Phospholipid** | **Concentration** | **Molecular weight** |
|---|---|---|---|
| **PC** | L-alpha-phosphatidylcholine | 10 or 25 mg/ml | 761 |
| **PS** | L-alpha-phosphatidylserine, bovine brain sodium salt | 10 mg/ml | 810 |
| **PE** | L-alpha-phosphatidylethanolamine, bovine liver | 10 mg/ml | 768 |

### Buffers:

| **HBS** |
|---|
| 100 mM NaCl |
| 20 mM Hepes/NaOH, pH 7.5 |
| 0.02 % (m/v) sodium azide |
| **HBSA (keep at 4 °C)** |
| Bovine serum albumin in 0.1 % (m/v) HBS |
| **OG/HBS (prepare at time of use)** |
| n-octyl-beta-D-glucopyranoside in 100 mM HBS (29.2 mg OG/ ml of HBS) |

### Preparing the phospholipid solution in octylglucoside (OG)

1. 2.6 micromols of total phospholipids are prepared for each sample in a glass tube, using the desired phospholipid molar radius.
2. Dry the phospholipid mixture under an argon or nitrogen current.
3. When the tube appears to be dry, vacuum dry another 60 minutes.
4. Add 400 µl of a fresh solution of OG/HBS (at room temperature) to the tube containing the dry phospholipids.

| | | |
|---|---|---|
| **For PC:PS vesicles (80:20 molar ratio)** | | |
| 62 µl PC (at 25 mg/ml) . | = 1.58 mg | = 2.08 µmol |
| 42 µl PS (at 10 mg/ml) | = 0.42 mg | = 0.52 µmol |

| **For PC:PE:PC vesicles (40:40:20 molar ratio)** | | |
|---|---|---|
| 32 µl PC (at 25 mg/ml) | = 0.79 mg | = 1.04 µmol |
| 80 µl PE (at 10 mg/ml) | = 0.80 mg | = 1.04 µmol |
| 42 µl PS (at 10 mg/ml) | = 0.42 mg | = 0.52 µmol |

### Relipidating

Add the desired amount of TF to the tube containing the 400 µl of OG/phospholipids and enough HBSA up to completing the final volume of 1 ml. Carry out this step at room temperature.

### Obtaining washed platelet suspensions

Washed platelet suspensions were prepared according to the method described by Radomski M. et al. (Radomski M, Moncada S.; 1983 Thromb Res. An improved method for washing of human platelets with prostacyclin. 15;30(4):383-9) from blood extractions (3.15% sodium citrate) from healthy volunteers. Processing of the specimens was always carried out immediately after blood extraction and at room temperature. Platelet activation and functionality states were assayed by means of aggregation assays prior to and during the performance of the assays. Self-activation and functionality were estimated by means of activation with a known agonist (collagen).

### Platelet aggregation assays

Platelet aggregation assays were performed in washed platelets to prevent interference from plasma coagulation factors, obtained following the previously described protocol. These assays were performed by means of an optical method, following the aggregometer handling protocol (Chromogenix). All the information was processed by the Aggro/link interface which was recorded on a Pentium III computer. In short, 500 µl of washed platelets were added in the cuvettes along with the stirring magnet and they were tempered for 1 minute at 37°C. They were arranged in the aggregometer along with a cuvette for the blank (platelet suspension buffer), the reagents to be analyzed were added in two of the channels and a reference reagent in the third, and aggregation time was recorded.

### In vitro assays

### Chromogenic assays

Different chromogenic assays were designed in solution and using activated platelet suspensions as a procoagulant surface to demonstrate the effect of rTF on factor Xa proteolytic activity.

FXa amidolytic activity was determined by means of a chromogenic assay using S-2765 (Chromogenix) as the chromogenic substrate for FXa, whereas thrombin forming activity was analyzed using S-2238 (Chromogenix) as the chromogenic substrate for thrombin.

The chromogenic assays were performed in suspension (in buffer) and using washed platelet suspensions. Suitable volumes of each one of the factors to be studied were dispensed on an ELISA plate and, in the event of using the washed platelet suspension, a suitable volume such as to have a concentration of 250,000 platelets/µl in the medium. Finally, adding the specific chromogenic substrate allowed quantifying the proteolytic activity in question by means of spectrophotometric readings at 405 nm. In the assays for determining amidolytic activity only FXa and the different recombinant Tissue factor (rTF) concentrations were dispensed, whereas in the assays for determining thrombin forming activity it was necessary to further add factor II (prothrombin) and factor Va (the latter only in the case of assays in suspension, given that in the washed platelet assays these already contained endogenous FVa).

### Coagulation assays in plasma

Spontaneous procoagulant activity (unstimulated) in plasma was measured by means of a coagulation assay with a certain step in a coagulometer (Fibrintimer BFT-II clot-timer Dade-Behring, Germany). In short, 50 µl of platelet-poor plasma were added to the already tempered cuvettes and 50 µl of distilled water were added. This mixture was left to incubate for 60 seconds at 37°C and 50 µl of 25 mM calcium chloride were immediately added and the coagulation time was determined in seconds in the coagulometer, verified by formation of the clot. Each one of the samples was assayed in duplicate.

### Coagulation assays in whole blood

Procoagulant activity in non-anticoagulated whole blood was determined by means of a coagulation method. The different agents to be studied were added to 1 ml of non-anticoagulated whole blood and coagulation time was measured with a chronometer from the beginning of the extraction until a stable and consolidated blood clot appeared. The effect of the different agents was evaluated by means of their shortening or lengthening of blood coagulation times.

### In vivo assays

### Severe hemorrhage model by rat tail proximal section

28 Sprage-Dawle male rats weighing 350-450 grams were randomly distributed in 4 treatment groups: a control group, made up of 7 animals which received topical treatment with physiological saline solution, whereas the other three groups, also made up of 7 animals, received topical treatment with 10 µg/ml rTF, topical treatment with 10 µg/ml rTF + 100 µl of the reagent containing the procoagulant inorganic surface (Dade® Actin® FS (Dade Behring)) and topical treatment with 10 µg/ml rTF + FXa at a concentration of 400 ng/ml, respectively. All the compounds came into topical contact with the proximal section of the animal's tail to hemostatically act in a volume of 1 ml dispensed in a well of a 6-well ELISA plate. Formation of the stable and consolidated clot was evidenced by means of confirmation of no further bleeding.

### Lethal hemorrhage model by puncture in the carotid artery of rats

12 male Sprage-Dawle rats weighing 350-450 grams were randomly distributed in 4 treatment groups each including 3 animals. The control group animals received physiological saline solution as treatment, whereas the other three groups received rTF alone or combined with the procoagulant inorganic surface and FXa. The compounds and the physiological saline solution were administered directly on the point of the puncture in a volume of 5 ml with an automatic pipette (several cycles) combined with the pressure and contact of a dressing containing the corresponding treatment. The group treated with rTF (alone) received 10 µg/ml and the corresponding dressing on the puncture also containing 10 µg/ml rTF. The group treated with rTF and inorganic surface received 5 ml of a solution formed by 2.5 ml of 10 µg/ml rTF and 2.5 ml of surface as well as a dressing on the puncture containing the same composition. Finally, the group treated with rTF and FXa also received 5 ml of a solution formed by 2.5 ml of 10 µg/ml rTF and 2.5 ml of 400 ng/ml FXa.

### II. RESULTS

### II.1 In vitro assays

### A. TF increases FXa proteolytic activity and accordingly thrombin production

The following *in vitro* assays were performed for the purpose of evaluating TF capacity as a factor Xa stimulating agent in the absence of FVII: (i) chromogenic assays for FXa amidolytic activity in solution and in washed platelet suspension; and (ii) chromogenic assays for thrombin forming activity in solution and in washed platelet suspension.

### A.1 Chromogenic assays for FXa amidolytic activity

### A.1.1 Assays in solution

Direct assays for FXa amidolytic activity in solution using FXa-specific substrate S-2765, demonstrated that rTF is able to very significantly increase FXa proteolytic activity. In the presence of high rTF concentrations (10 µg/ml) an increase of 4-6 times the activity produced by low and high FXa concentrations (p<0.001) was observed. Similar significant stimulating effects (p<0.001) were observed in the presence of lower rTF concentrations. Table 1 shows the results obtained in 5 independent experiments.

**Table 1**

| | | | | | |
|---|---|---|---|---|---|
| **rTF, in the absence of FVII, increases FXa proteolytic activity Chromogenic assay with FXa-specific substrate (Substrate S-2765 amidolytic activity)** | | | | | |
| | Without rTF | rTF 10 µg/ml | rTF 1 µg/ml | rTF 0.1 µg/ml | rTF 0.01 µg/ml |
| FXa 4 µg/ml | 550 ± 51 | 3,316 ± 89 | 2,108 ± 61 | 1,275 ± 24 | 801 ± 36 |
| FXa 0.4 µg/ml | 150 ± 32 | 505 ± 63 | 341 ± 32 | 194 ± 15 | 203 ± 11 |

| | | | | | |
|---|---|---|---|---|---|
| *Mean ± SEM (n= 5)* | | | | | |

### A.1.2 Assays in washed platelet suspension

Similar results were obtained when using washed platelet suspensions as a source of procoagulant surface. In the presence of very low exogenous FXa concentrations, rTF caused a significant stimulating effect on the amidolytic activity evaluated with specific substrate S-2765 (p<0.001). Similar stimulating effects were observed in the presence of high FXa concentrations (Table 2).

**Table 2**

| **TF induces FXa amidolytic activity (S-2765) expressed in washed platelets** | | | | |
|---|---|---|---|---|
| | **Without rTF** | **rTF 10 µg/ml** | **rTF 1 µg/ml** | **rTF 0.1 µg/ml** |
| FXa 0.4 µg/ml | 220 ± 12 | 706 ± 32 | 655 ± 41 | 291 ± 33 |
| FXa 80 ng/ml | 26 ± 3 | 289 ± 11 | 311 ± 22 | 165 ± 28 |
| FXa 40 ng/ml | 9 ± 2 | 341 ± 14 | 306 ± 19 | 143 ± 14 |

| | | | | |
|---|---|---|---|---|
| *Mean ± SEM (n= 5)* | | | | |

### A.2 Chromogenic assays for thrombin forming activity

### A.2.1 Assays in solution

Similarly to that observed in reference to FXa amidolytic activity, indirect assays for thrombin forming activity in solution using S-2238 as the specific substrate demonstrated that rTF is able to very significantly increase thrombin forming activity from hydrolysis of prothrombin (FII). FXa was able to form thrombin only in the presence of FII and FVa, and this production was significantly increased in the presence of rTF (p<0.001). Table 3 shows the results obtained from 5 independent experiments.

**Table 3**

| **rTF increases FXa thrombin forming activity (substrate S 2238) Chromogenic assay with specific substrate for thrombin in the presence and absence of FII and FVa** | | | | |
|---|---|---|---|---|
| | **Without rTF** | **rTF 10 µg/ml** | **rTF 0.1 µg/ml** | **rTF 0.01 µg/ml** |
| FXa 4 µg/ml | 0 | 0 | 0 | 0 |
| FXa 4 µg/ml + FII | 0 | 0 | 0 | 0 |
| FXa 4 µg/ml + FII + FVa | 305 ± 120 | 5,850 ± 142 | 705 ± 45 | 612 ± 38 |

| | | | | |
|---|---|---|---|---|
| *Mean ± SEM (n= 5)* | | | | |

### A.2.2 Assays in washed platelet suspension

The procoagulant effect detected was much greater when platelet suspensions were used as the source of the procoagulant surface and thrombin formation was determined by means of specific substrate S-2238. In the absence of exogenous FXa, rTF produced a significant stimulating effect on thrombin formation (p<0.001). Similar stimulating effects were observed in the presence of exogenous FXa both at low and high concentrations (p<0.001). Table 4 shows the results obtained in five independent experiments.

**Table 4**

| **rTF induces thrombin formation (S-2238) in washed platelets in the presence of FII but in the absence of FVa (already present in platelets)** | | | | | |
|---|---|---|---|---|---|
| | **Without rTF** | **rTF 10 µg/ml** | **rTF 5 µg/ml** | **rTF 0.1 µg/ml** | **rTF 0.01 µg/ml** |
| FXa 80 ng/ml | 902 ± 25 | 13,750 ± 145 | 11,955 ± 89 | 12,150 ± 102 | 3,501 ± 65 |
| FXa 40 ng/ml | 421 ± 19 | 14,910 ± 168 | 13,450 ± 198 | 13,854 ± 145 | 2,906 ± 56 |
| FXa 4 ng/ml | 213 ± 21 | 9,610 ± 114 | 8,951 ± 132 | 9,324 ± 155 | 1,954 ± 124 |
| FXa 0.8 ng/ml | 201 ± 29 | 8,508 ± 178 | 8,105 ± 210 | 7,802 ± 113 | 2,102 ± 89 |
| FXa absent | 0 | 3,504 ± 69 | 1,908 ± 57 | 906 ± 89 | 613 ± 42 |

| | | | | | |
|---|---|---|---|---|---|
| *Mean ± SEM (n= 5)* | | | | | |

### B. Platelet aggregation assays

The experiments for determining if rTF could induce platelet aggregation and if this was mediated by interaction with platelet-transported and exposed FXa were designed from the experiments demonstrating that rTF is able to induce thrombin formation in platelet suspensions in the absence of exogenous FXa. Table 5 lists the assay results in which rTF in the absence of exogenous FXa is demonstrated to be able to induce platelet aggregation and that this is mediated by FXa through signaling of its receptor, membrane receptor EPR-1. The inhibiting effect achieved with monoclonal antibodies directed against both FXa and receptor EPR-1 demonstrates that rTF interacts with FXa exposed on the platelet surface, which mediates platelet aggregation by means of the action of this proteolytic enzyme, and that said aggregation is signaled by the FXa-specific receptor, EPR-1.

Accordingly, formation of the TF:FXa complex on the platelet surface, as well as producing thrombin formation, produces platelet aggregation and further enhances hemostatic clot formation.

**Table 5**

| | | | | | |
|---|---|---|---|---|---|
| **rTF induces platelet aggregation mediated by FXa through the signaling of its receptor (EPR-1)** | | | | | |

| | **rTF concentration [ng/ml]** | | | | |
|---|---|---|---|---|---|
| | **Without rTF** | **5** | **10** | **100** | **250** |
| Without FXa | 0 | 80 | 100 | 100 | 100 |
| FXa 8 pg/ml | 0 | 100 | 100 | 100 | 100 |
| FXa 80 pg/ml | 0 | 100 | 100 | 100 | 100 |
| FXa 240 pg/ml | 100 | 100 | 100 | 100 | 100 |
| anti FXa (24 µg/ml) + FXa 240 pg/ml | 0 | | | 0 | |
| anti EPR-1 (20 µg/ml) + FXa 240 pg/ml | 0 | | | 0 | |

| | | | | | |
|---|---|---|---|---|---|
| *Mean ± SEM (n= 5)* | | | | | |

### C. rTF is able to coagulate plasma and blood in healthy subjects and in FVII-deficient patients

A series of *in vitro* coagulation assays were performed showing the coagulating effect of rTF in plasma and non-anticoagulated whole blood in healthy subjects and in FVII-deficient patients and, therefore, rTF is a useful agent for antihemorrhagic treatment of said individuals. The currently existing hemorrhage treatment in FVII-deficient patients consists in the administration of recombinant FVII (FVII).

### C.1 Coagulation assays in plasma

The effect of rTF on plasma coagulation was investigated by means of coagulation assays using plasmas from healthy volunteers without a history of hemostatic disorders (bleeding or thrombosis) and the plasma from 2 patients with FVII-deficiency.

rTF was able to very significantly accelerate, and in a concentration-dependent manner, spontaneous plasma coagulation induced by recalcification of a plasma sample previously anticoagulated with sodium citrate. Surprisingly, the plasma from the 2 FVII-deficient patients were coagulated in the presence of low, medium and high rTF concentrations, indicating that it is able to produce plasma coagulation even in the absence of FVII, rTF. Table 6 shows the results obtained from 5 independent experiments for the normal plasmas and 2 for the FVII-deficient plasmas.

**Table 6**

| | | | | |
|---|---|---|---|---|
| **Demonstration of the procoagulant effect of TF in normal and FVII-deficient plasmas** | | | | |

| | **Coagulation time (s)** | | | |
|---|---|---|---|---|
| | **Without rTF** | | **With rTF** | |
| | - | 100 µg/ml | 10 µg/ml | 1 µg/ml |
| Normal plasma (5 mM Ca) | 215.1 ± 24.6 | 11.1 ± 0.2 | 15.3 ± 0.2 | 25.7 ± 0.4 |
| FVII-deficient plasma | > 300 | 67.7 ± 7.2 | 113.7 ± 28.3 | 202.7 ± 41.6 |

### C.2 Coagulation assays in non-anticoagulated whole blood

The procoagulant effect of rTF in human non-anticoagulated whole blood from healthy individuals and FVII-deficient patients was evaluated by means of a coagulation assay. Formation of the clot was determined by estimating the time in minutes for the clot to consolidate. The effect of rTF on blood from healthy volunteer was significant after the concentration of 0.1 µg/ml, whereas greater concentrations were necessary in FVII-deficient patients, significant procoagulant effects (p<0.001) being detected after 1 µg/ml. rTF was able to completely normalize coagulation time at the concentration of 10 µg/ml, there being no differences between coagulation times detected in normal subjects and FVII-deficient subjects with said rTF concentration. Table 7 shows the results obtained from 4 samples from healthy individuals and 2 from FVII-deficient patients.

**Table 7**

| **Procoagulant effect of rTF in non-anticoagulated whole blood from healthy and FVII-deficient individuals** | | | | | | |
|---|---|---|---|---|---|---|
| | **Basal** | | | **rTF** | | |
| | | 0.01 µg/ml | 0.1 µg/ml | 1 µg/ml | 10 µg/ml | 100 µg/ml |
| Sample no. 1 | 5.8 | 4.7 | 4.4 | 3.3 | 2.1 | 1.3 |
| Sample no. 2 | 7.2 | 6.7 | 5.5 | 3.7 | 2.1 | 1.0 |
| Sample no. 3 | 7.2 | 6.6 | 6.3 | 4.2 | 2.1 | 0.9 |
| Sample no. 4 | 7.5 | 6.7 | 5.7 | 3.8 | 2.0 | 1.0 |
| Patient no. 6 FVII-deficient | 11.4 | --- | 10.5 | 9.5 | 5.2 | 2.3 |
| Patient no. 7 FVII-deficient | 12.5 | --- | 11.3 | 10.5 | 6.3 | 3.1 |

### D. rTF produces blood coagulation in the presence of low FXa concentrations which on their own are unable to induce coagulation

*In vitro* coagulation assays were performed in plasma and in non-anticoagulated whole blood which showed the synergistic effect of rTF on FXa (used at low concentrations which on their own are unable to induce coagulation).

### D.1 Coagulation assays in plasma

The procoagulant effects of the association of rTF and FXa at low concentrations (80 pg/ml) unable to induce coagulation on their own were investigated in normal plasma (Table 8) and in FVII-deficient plasma (Table 9). The procoagulant effect observed exceeded that obtained when rTF was used as a single procoagulant agent. Even at the lowest rTF concentrations (0.001 µg/ml) at which rTF was not able to coagulate the FVII-deficient plasma, the combination of both agents produced a significant procoagulant effect.

**Table 8**

| **Procoagulant effect of rTF associated with FXa (80 pg/ml) in normal plasma** | | |
|---|---|---|
| **Normal Plasma** | **Coagulation time (s)** | |
| | Without FXa | With FXa (80 pg/ml) |
| Basal (5 mM calcium) | 281.08 ± 12.5 | 206 ± 18 |
| rTF 0.001 µg/ml | 201 ± 12.2 | 46 ± 2.4* |
| rTF 0.01 µg/ml | 184 ± 5.8 | 24.6 ± 1.9* |
| rTF 0.1 µg/ml | 73.5 ± 2.3 | 17.4 ± 3.3* |
| rTF 1 µg/ml | 28.6 ± 0.1 | 12.6 ± 3.2* |
| rTF 10 µg/ml | 16.4 ± 0.9 | 9.1 ± 0.4* |
| rTF 100 µg/ml | 10.8 ± 0.3 | 6.6 ± 0.2* |

| | | |
|---|---|---|
| *Mean ± SEM (n = 5); *p<0.001 without FXa vs. FXa. t -Student* | | |

**Table 9**

| **Procoagulant effect of TF associated with FXa (80 pg/ml) in FVII-deficient plasma** | | | |
|---|---|---|---|
| | **Coagulation time (s)** | | |
| | **Without rTF** | **With rTF** | |
| | | 1 µg/ml | (1 µg/ml) + FXa 80 pg/ml |
| Normal plasma | 281.1 ±12.5 | 28.6 ± 0.1 | 12.6 ± 3.2 |
| FVII-D Plasma | > 300 | 202.7 ± 41.6 | 14.8 ± 0.2* |

| | | | |
|---|---|---|---|
| *Mean ± SEM (n=5); * p<0.001 without FXa vs. FXa. t -Student* | | | |

### D.2 Coagulation assays in human non-anticoagulated whole blood

The procoagulant effect in non-anticoagulated whole blood of the association of rTF and FXa at low concentrations (80 pg/ml) in blood from healthy individuals and from FVII-deficient patients greatly exceeded that obtained when rTF was used as a single procoagulant agent. Even at the lowest rTF concentrations (0.001 µg/ml) at which rTF was not able to coagulate the whole blood of normal individuals, the combination of both agents produced a significant procoagulant effect. Likewise, similar potent synergistic effects were observed when the blood of FVII-deficient patients was used, allowing to use much lower rTF concentrations to obtain greater procoagulant effects. In the absence of rTF, the FXa concentrations used were unable to produce any procoagulant effect, indicating that when FXa is present at very low concentrations it requires the presence of TF for the enzyme to exert its proteolytic effect. Table 10 shows the results obtained in 5 independent experiments with whole blood samples from healthy individuals, whereas Table 11 shows the results obtained in 2 FVII-deficient patients.

**Table 10**

| **Procoagulant effect of rTF together with FXa (80 pg/ml) in healthy individuals** | | |
|---|---|---|
| | **Without FXa** | **With FXa (80 pg/ml)** |
| Basal (5 mM calcium) | 7.1 ± 0.6 | 7.05 ± 0.9 |
| rTF 0.01 µg/ml | 6.1 ± 1.5 | 4.8 ± 1.1* |
| rTF 0.1 µg/ml | 5.1 ± 1.9 | 3.8 ± 1.3* |
| rTF 1 µg/ml | 3.2 ± 0.1 | 2.4 ± .0.2* |
| rTF 10 µg/ml | 2.1 ± 0.9 | 1.8 ± 0.1* |
| rTF 100 µg/ml | 1.1 ± 0.1 | 0.5 ± 0.1* |

| | | |
|---|---|---|
| *Coagulation time (expressed in minutes): the time the clot takes to consolidate in a non-anticoagulated blood sample* *Mean ± SEM (n = 5)* p<0.001 without FXa vs. FXa.t―Student* | | |

**Table 11**

| **Procoagulant effect of rTF together with FXa (80 pg/ml) in FVII-deficient patients** | | | |
|---|---|---|---|
| | **Basal** | **rTF 1 µg/ml** | **rTF 1 µg/ml + FXa 80 pg/ml** |
| FVII-deficient patient | 11.4 | 9.5 | 2.4* |
| FVII-deficient patient | 12.5 | 10.5 | 1.4* |

| | | | |
|---|---|---|---|
| *Coagulation time (expressed in minutes): the time the clot takes to consolidate in a non-anticoagulated blood sample* *Mean ± SEM (n = 5) * p<0.001 without FXa vs. FXa.t-Student* | | | |

### E. rTF (alone and associated with low FXa concentrations) coagulates plasma and blood from healthy subjects and patients with deficiencies in coagulation factors different to FVII

A series of *in vitro* coagulation assays were performed showing the coagulant effect of rTF (alone and associated with low FXa concentrations) in plasma and non-anticoagulated whole blood of patients with deficiencies in coagulation factors different to FVII (FV, FVIII, FIX, FXI and FXII) and A and B hemophilic patients; therefore, rTF is a useful agent for the antihemorrhagic treatment of said individuals.

### E.1 Coagulation assays in coagulation factor-deficient plasma

### E.1.1 Effect of rTF (alone)

The procoagulant effect of rTF (alone) on coagulation factor-deficient plasma was investigated using commercial plasma depleted by means of immunoaffinity techniques, as well as plasma from 3 patients diagnosed with hemophilia A (FVIII-deficient) and 2 diagnosed with hemophilia B (FIX-deficient). Table 12 shows the results. At the concentration of 100 µg/ml rTF was able to effectively coagulate all the plasmas with deficiencies in FV, FVIII, FIX, FXI and FXII. On the other hand, in the absence of the known FXa cofactor, FVa, TF was also able to produce plasma coagulation, although only at high and medium concentrations. In the remaining coagulation factor deficiencies, rTF was effective at all the concentrations used, with excellent results being obtained at very low concentrations. These results were confirmed when samples from patients suffering congenital coagulopathies, such as hemophilia A and hemophilia B. rTF was able to coagulate the plasmas previously incubated with high heparin concentrations, demonstrating that its effect on FXa is independent of the inhibitory effect mediated by this anticoagulant drug and, even in the presence of heparin-type inhibitors, rTF is effective in producing plasma coagulation.

**Table 12**

| **Procoagulant effect of TF (alone) in heparinized and coagulation factor-deficient plasmas** | | | | |
|---|---|---|---|---|
| | **Coagulation time (s)** | | | |
| | **Without rTF** | | **With rTF** | |
| | - | 100 µg/ml | 10 µg/ml | 1 µg/ml |
| Normal plasma | 215.1 ± 24.6 | 11.1 ± 0.2 | 15.3 ± 0.2 | 25.7 ± 0.4 |
| FV-D plasma | > 300 | 84.3 ± 15.2 | 123.3 ± 24.5 | > 300 |
| FVIII-D plasma | > 300 | 15.5 ± 2.3 | 21.5 ± 2.8 | 32.1 ± 2.6 |
| FIX-D plasma | > 300 | 14.8 ± 3.2 | 22.3 ± 2.5 | 34.6 ± 2.4 |
| FXI-D plasma | > 300 | 14.3 ± 0.5 | 19.4 ± 1.5 | 27.3 ± 0.6 |
| FXII-D plasma | > 300 | 16.2 ± 0.3 | 22.5 ± 1.1 | 32.5 ± 0.5 |
| Hemophilia A plasma | > 300 | 12.2 ± 1.5 | 13.8 ± 0.6 | 21.3 ± 0.8 |
| Hemophilia B plasma | > 300 | 12.1 ± 0.5 | 15.3 ± 0.4 | 26.8 ± 0.6 |
| 10µg/ml heparin plasma | > 300 | 20.3 ± 0.6 | 28 ± 0.8 | 36.7 ± 2.3 |

| | | | | |
|---|---|---|---|---|
| *Mean* ± *SEM (n = 5)* | | | | |

### E.1.2 Effect of the rTF + FXa combination at low concentrations

Likewise, the procoagulant effect of the rTF and FXa combination at low concentrations (80 pg/ml) in normal plasmas and in the different coagulation factor-deficient plasmas (FV, FVIII, FIX, FXI and FXII), was greater than that obtained when rTF was used alone as the procoagulant agent (Table 13). Even at the lowest rTF concentrations (0.001 µg/ml), with which rTF was not able to coagulate the FV-deficient plasma, the combination of both agents (rTF + FXa) produced a significant procoagulant effect.

**Table 13**

| **Procoagulant effect of TF associated with FXa (80 pg /ml) in heparinized and coagulation factor-deficient plasmas** | | | |
|---|---|---|---|
| | **Coagulation time (s)** | | |
| | **Without rTF** | | **With rTF** |
| | | 1 µg/ml | (1 µg/ml) + FXa 80 pg/ml |
| Normal plasma | 281.1 ± 12.5 | 28.6 ± 0.1 | 12.6 ± 3.2 |
| FV-D plasma | > 300 | > 300 | 87.2 ± 1.2* |
| FVIII-D plasma | > 300 | 32.1 ± 2.6 | 12.8 ± .0.9* |
| FIX-D plasma | > 300 | 34.6 ± 2.4 | 15.6 ± 1.6* |
| FXI-D plasma | > 300 | 27.3 ± 0.6 | 14.9 ± 2.7* |
| FXII-D plasma | > 300 | 32.5 ± 0.5 | 12.4 ± 2.9* |
| Hemophilia A plasma | > 300 | 21.3 ± 0.8 | 10.9 ± 0.5* |
| Hemophilia B plasma | > 300 | 26.8 ± 0.6 | 11.3 ± 0.9* |
| 10µg/ml heparin plasma | > 300 | 36.7 ± 2.3 | 12.3 ± 0.7* |

| | | | |
|---|---|---|---|
| *Mean ± SEM (n = 5); *p<0.001 without FXa vs. FXa. t -Student* | | | |

### E.2 Coagulation assays in human blood from hemophilia A and B patients

### E.2.1 Effect of rTF (alone)

The procoagulant effect of rTF (alone) in human non-anticoagulated whole blood from people suffering from hemophilia A and B, was evaluated by means of a coagulation assay. Clot formation was determined by estimating the time in minutes for the clot to consolidate. The effect of rTF in blood from healthy volunteers was significant from concentrations of 0.1 µg/ml, whereas greater doses were required in hemophilic patients, significant procoagulant effects (p<0.001) being detected from 1 µg/ml. At the concentration of 10 µg/ml rTF was able to completely normalize coagulation time, there being no differences between the coagulation times detected in normal subjects and hemophilic subjects. Table 14 shows the results obtained from 4 samples from healthy individuals (sample numbers 1-4), 3 from patients suffering hemophilia A and 2 with hemophilia B.

**Table 14**

| **Procoagulant effect of rTF in non-anticoagulated whole blood** | | | | | | |
|---|---|---|---|---|---|---|
| | **Basal** | | | **rTF** | | |
| | | 0.01 µg/ml | 0.1 µg/ml | 1 µg/ml | 10 µg/ml | 100 µg/ml |
| Sample no. 1 | 5.8 | 4.7 | 4.4 | 3.3 | 2.1 | 1.3 |
| Sample no. 2 | 7.2 | 6.7 | 5.5 | 3.7 | 2.1 | 1.0 |
| Sample no. 3 | 7.2 | 6.6 | 6.3 | 4.2 | 2.1 | 0.9 |
| Sample no. 4 | 7.5 | 6.7 | 5.7 | 3.8 | 2.0 | 1.0 |
| Patient no. 1 Hemophilia A | 13.3 | --- | 13.0 | 8.0 | 4.6 | 2.6 |
| Patient no. 2 Hemophilia A | 17.3 | --- | 16.5 | 9.0 | 5.6 | 3.0 |
| Patient no. 3 Hemophilia A | 15.3 | --- | 15.2 | 12.4 | 7.0 | 4.5 |
| Patient no. 4 Hemophilia B | 20.5 | --- | 19.4 | 11.1 | 6.1 | 3.5 |
| Patient no. 5 Hemophilia B | 16.3 | --- | 16.0 | 11.3 | 5.5 | 3.6 |

| | | | | | | |
|---|---|---|---|---|---|---|
| *Coagulation time (expressed in minutes): the time the clot takes to consolidate in a non-anticoagulated blood sample* | | | | | | |

### E.2.2 Effect of the rTF + FXa combination at low concentrations

Likewise, the procoagulant effect in non-anticoagulated whole blood of the rTF and FXa combination at low concentrations (80 pg/ml) in blood from healthy individuals and from patients with hemophilia A and B greatly exceeded that obtained when only rTF was used as the single procoagulant agent. Even at the lowest rTF concentrations (0.001 µg/ml), with which rTF was not able to coagulate the whole blood of normal individuals, the combination of both agents produced a significant procoagulant effect. Likewise, similar potent synergistic effects were observed when using the blood of hemophilic patients, allowing the use of much lower rTF concentrations to obtain greater procoagulant effects. In the absence of rTF, the FXa concentrations used were unable to produce any procoagulant effect at all, indicating that when FXa is present at very low concentrations it requires the presence of TF for the enzyme to exert its proteolytic action. Tables 15 and 16 show the results obtained in 5 independent experiments with whole blood samples from healthy individuals (Table 15) and the blood samples from 3 patients with hemophilia A and 2 patients with hemophilia B (Table 16).

**Table 15**

| **Procoagulant effect of TF together with FXa (80 pg/ml)** | | |
|---|---|---|
| | **Without FXa** | **With FXa (80 pg/ml)** |
| Basal (5 mM calcium) | 7.1 ± 0.6 | 7.05 ± 0.9 |
| rTF 0.01 µg/ml | 6.1 ± 1.5 | 4.8 ± 1.1* |
| rTF 0.1 µg/ml | 5.1 ± 1.9 | 3.8 ± 1.3* |
| rTF 1 µg/ml | 3.2 ± 0.1 | 2.4 ± .0.2* |
| rTF 10 µg/ml | 2.1 ± 0.9 | 1.8 ± 0.1* |
| rTF 100 µg/ml | 1.1 ± 0.1 | 0.5 ± 0.1* |

| | | |
|---|---|---|
| *Coagulation time (expressed in minutes): the time the clot takes to consolidate in a non-anticoagulated blood sample* *Mean ± SEM (n = 5); *p<0.001 without FXa vs. FXa. t -Student* | | |

**Table 16**

| **Procoagulant effect of TF together with FXa (80 pg/ml) in hemophilic patients** | | |
|---|---|---|
| | **Basal** | **rTF 1 µg/ml + FXa 80 pg/ml** |
| Hemophilia A | 13.3 | 2.5* |
| Hemophilia A | 17.3 | 2.8* |
| Hemophilia A | 15.3 | 2.5* |
| Hemophilia B | 20.5 | 1.9* |
| Hemophilia B | 16.3 | 1.8* |

| | | |
|---|---|---|
| *Coagulation time (expressed in minutes): the time the clot takes to consolidate in a non-anticoagulated blood sample* *Mean ± SEM (n = 5); *p<0.001 without FXa vs. FXa. t -Student* | | |

### F. rTF coagulates the blood of patients suffering from platelet disorders

A series of *in vitro* coagulation assays were performed showing the coagulant effect of rTF in non-anticoagulated whole blood of patients suffering from platelet disorders; rTF is therefore a useful agent for the antihemorrhagic treatment of said individuals.

### F.1 Coagulation assays in non-anticoagulated whole blood of patients with platelet disorders

The procoagulant effect of rTF on blood coagulation in patients with platelet disorders was investigated by means of coagulation assays using blood from 2 patients suffering Glanzmann's disease and Bernard Soulier syndrome.
rTF was able to very significantly accelerate, and in a concentration-dependent manner, blood coagulation both in the healthy individuals (sample numbers 1-4) and in individuals suffering from Glanzmann's disease and Bernard Soulier syndrome (Table 17).

**Table 17**

| **Procoagulant effect of TF in patients with platelet disorders** | | | | | | |
|---|---|---|---|---|---|---|
| | **Basal** | | | **rTF** | | |
| | | 0.01 µg/ml | 0.1 µg/ml | 1 µg/ml | 10 µg/ml | 100 µg/ml |
| Sample no. 1 | 5.8 | 4.7 | 4.4 | 3.3 | 2.1 | 1.3 |
| Sample no. 2 | 7.2 | 6.7 | 5.5 | 3.7 | 2.1 | 1.0 |
| Sample no. 3 | 7.2 | 6.6 | 6.3 | 4.2 | 2.1 | 0.9 |
| Sample no. 4 | 7.5 | 6.7 | 5.7 | 3.8 | 2.0 | 1.0 |
| Patient no. 6 Glanzmann's dis. | 13.6 | --- | 10.2 | 7.3 | 3.4 | 2.9 |
| Patient no. 7 Bernard-Soulier S. | 11.9 | --- | 9.3 | 7.1 | 4.1 | 3.1 |

### G. rTF, associated with negatively charged inorganic surfaces, coagulates plasma and blood from healthy subjects and coagulation factor-deficient patients

### G.1 Coagulation assays in plasma from healthy subjects

### Effect of the rTF and Dade® Actin® FS reagent (Dade Behring) combination

A series of *in vitro* coagulation assays were performed showing the coagulant effect of rTF associated with negatively charged inorganic surfaces [NCIS] (Dade® Actin® FS reagent) in plasma from healthy volunteers without histories of hemostatic disorders. A constant concentration of 100 µl of the Dade® Actin® FS reagent was used in all the combination assays, whereas the rTF concentration was varied. Table 18 shows the results obtained from 5 independent experiments. By comparison with the assays in which rTF was alone, the procoagulant effect of the association (rTF + NCIS) was significantly (p<0.001) greater in each and every one of the concentrations used.

**Table 18**

| **Procoagulant effect of rTF associated with negatively charged inorganic surfaces (NCIS) in normal plasma** | | |
|---|---|---|
| **Normal plasma** | **Coagulation time (s)** | |
| | Without surface | With surface (100 µl) |
| Basal (5 mM calcium) | 281.08 ± 12.5 | 167 ± 10 * |
| rTF 0.001 µg/ml | 201 ± 12.2 | 69 ± 0.8* |
| rTF 0.01 µg/ml | 184 ± 5.8 | 44.9 ± 3.2 * |
| rTF 0.1 µg/ml | 73.5 ± 2.3 | 30.6 ± 2.5* |
| rTF 1 µg/ml | 28.6 ± 0.1 | 16 ± 4.2* |
| rTF 10 µg/ml | 16.4 ± 0.9 | 12.8 ± 0.5* |
| rTF 100 µg/ml | 10.8 ± 0.3 | 7.2 ± 0.3* |

| | | |
|---|---|---|
| *Mean ± SEM (n = 5); *p<0.001 without NCIS vs. with NCIS; t-Student* | | |

### G.2 Coagulation assays in coagulation factor-deficient plasma

### Effect of the rTF and Dade® Actin® FS reagent combination

A series of *in vitro* coagulation assays were performed showing the coagulant effect of rTF associated with negatively charged inorganic surfaces [NCIS] (Dade® Actin® FS reagent) in plasma from coagulation factor-deficient patients (FV, FVII, FVIII, FIX, FXI and FXII).

Said procoagulant effect exceeded the one obtained when rTF was used alone as a procoagulant agent. Even at low concentrations (1 µg/ml) with which rTF was not able to coagulate the FV-deficient plasma (FV-D plasma), the combination of both agents produced a significant procoagulant effect. Likewise, similar synergistic effects were obtained when FVII-deficient plasma (FVII-D plasma) was used. These results show that the effect mediated by rTF is independent of FVII and is able to induce plasma coagulation even in the absence of FV. Similarly, the combination (rTF + NCIS) also exerted potent procoagulant effects in plasma samples from hemophilic patients (A and B), as well as in plasma samples preincubated with heparin at high concentrations (10 µg/ml). Table 19 shows the results obtained in 5 independent experiments.

**Table 19**

| **Procoagulant effect of rTF associated with negatively charged inorganic surfaces in heparinized and coagulation factor-deficient plasmas** | | | |
|---|---|---|---|
| | **Coagulation time (s)** | | |
| | **Without rTF** | **With rTF** | |
| | - | 1 µg/ml | 1 µg/ml + NCIS |
| Normal plasma | 281.1 ± 12.5 | 28.6 ± 0.1 | 16 ± 4.2* |
| FV-D plasma | > 300 | > 300 | 155.2 ± 2.3* |
| FVII-D plasma | > 300 | 202.7 ± 41.6 | 65 ± 3.4* |
| FVIII-D plasma | > 300 | 32.1 ± 2.6 | 12.1 ± 3.5* |
| FIX-D plasma | > 300 | 34.6 ± 2.4 | 14.3 ± 0.2* |
| FXI-D plasma | > 300 | 27.3 ± 0.6 | 16.2 ± 1.9* |
| FXII-D plasma | > 300 | 32.5 ± 0.5 | 11.5 ± 0.6 * |
| Hemophilia A plasma | > 300 | 21.3 ± 0.8 | 12.9 ± 1.5* |
| Hemophilia B plasma | > 300 | 26.8 ± 0.6 | 14.8 ± 0.9* |
| Heparin 10 µg/ml plasma | > 300 | 36.7 ± 2.3 | 15.4 ± 0.9 * |

| | | | |
|---|---|---|---|
| *Mean ± SEM (n = 5); *p<0.001 without NCIS vs. with NCIS; t -Student* FVD: FV-deficient; FVIID: FVII-deficient; FVIIID: FVIII-deficient; FIXD: FIX-deficient; FXID: FXI-deficient; FXIID: FXII-deficient | | | |

### G.3 Coagulation assays in human non-anticoagulated whole blood

The procoagulant effect in non-anticoagulated whole blood of the rTF and negatively charged inorganic surface [NCIS] (Dade® Actin® FS reagent) combination was also investigated se by means of coagulation assays using blood from (i) healthy individuals without histories of hemostatic disorders, (ii) patients suffering hemophilias A (3 patients) and B (2 patients) and (iii) patients suffering platelet disorders (Bernard-Soulier syndrome (1 patient) and Glanzmann's disease (1 patient)). A constant concentration of 100 µl of the Dade® Actin® FS reagent was used in all the combination assays, whereas the rTF concentration was varied. Table 20 shows the results obtained from 5 independent experiments. By comparison with the assays in which rTF was alone, the procoagulant effect of the association (rTF + NCIS) was significantly (p<0.001) greater in each and every one of the concentrations used.

**Table 20**

| **Procoagulant effect of rTF together with negatively charged inorganic surfaces (NCIS)** | | |
|---|---|---|
| | **Without NCIS** | **With NCIS** |
| Basal (5 mM calcium) | 7.1 ± 0.6 | 6.0 ± 1.4 * |
| rTF 0.01 µg/ml | 6.1 ± 1.5 | 5.2 ± 0.8 * |
| rTF 0.1 µg/ml | 5.1 ± 1.9 | 4.1 ± 1.1 * |
| rTF 1 µg/ml | 3.2 ± 0.1 | 2.8 ± .0.3 * |
| rTF 10 µg/ml | 2.1 ± 0.9 | 1.9 ± 0.1 |
| rTF 100 µg/ml | 1.1 ± 0.1 | 1 ± 0.1 |

| | **Basal** | **rTF 1 µg/ml + Surface** |
|---|---|---|
| Patient no. 1 Hemophilia A | 13.3 | 6.8 * |
| Patient no. 2 Hemophilia A | 17.3 | 7.2 * |
| Patient no. 3 Hemophilia A | 15.3 | 9.1 * |
| Patient no. 4 Hemophilia B | 20.5 | 8.5 * |
| Patient no. 5 Hemophilia B | 16.3 | 8.6 * |
| Patient no. 6 Glanzmann's dis. | 13.6 | 4.7 * |
| Patient no. 7 Bernard-Soulier S. | 11.9 | 4.6 * |

| | | |
|---|---|---|
| *Coagulation time (expressed in minutes): the time the clot takes to consolidate in a non-anticoagulated blood sample* *Mean ± SEM (n = 5); * p<0.001 without FXa vs. FXa. t -Student* | | |

### H. rTF associated with negatively charged inorganic surfaces and with FXa coagulates healthy subject and plasma and blood from coagulation factor-deficient patients

### H.1 Coagulation assays in plasma from healthy subjects

### Effect of the rTF, Dade® Actin® FS reagent and FXa combination

A series of *in vitro* coagulation assays were performed showing the coagulant effect of rTF associated with negatively charged inorganic surfaces [NCIS] (Dade® Actin® FS reagent) and with FXa in plasma from healthy volunteers without histories of hemostatic disorders. A constant concentration of Dade® Actin® FS reagent (100 µl) and of FXa (80 pg/ml) was used in all the assays for the combination, whereas the rTF concentration was varied. Table 21 shows the results obtained from 5 independent experiments. By comparison with the assays in which rTF was alone, the procoagulant effect of the association (rTF + NCIS + FXa) was significantly (p<0.001) greater in each and every one of the concentrations used. On the other hand, the NCIS + FXa combination was found to be the most potent.

**Table 21**

| **Procoagulant effect of rTF associated with (i) negatively charged inorganic surfaces (NCIS), or (ii) with FXa, or (iii) with NCIS and FXa in normal plasma** | | | | |
|---|---|---|---|---|
| **Normal plasma** | | **Coagulation time (s)** | | |
| | Without NCIS | With NCIS (100 µl) | With FXa (80 pg/ml) | With NCIS (100 µl) + FXa 80 pg/ml |
| Basal (5 mM calcium) | 281.08 ± 12.5 | 167 ± 10* | 206 ± 18 | 116 ± 14* |
| rTF 0.001 µg/ml | 201 ± 12.2 | 69 ± 0.8* | 46 ± 2.4* | 31 ± 3* |
| rTF 0.01 µg/ml | 184 ± 5.8 | 44.9 ± 3.2 * | 24.6 ± 1.9* | 16.1 ± 2* |
| rTF 0.1 µg/ml | 73.5 ± 2.3 | 30.6 ± 2.5* | 17.4 ± 3.3* | 11.2 ± 3* |
| rTF µg/ml | 28.6 ± 0.1 | 16 ± 4.2* | 12.6 ± 3.2* | 8.2 ± 1* |
| rTF 10 µg/ml | 16.4 ± 0.9 | 12.8 ± 0.5* | 9.1 ± 0.4* | 6.4 ± 0.5* |
| rTF 100 µg/ml | 10.8 ± 0.3 | 7.2 ± 0.3* | 6.6 ± 0.2* | 5.3 ± 0.1* |

| | | | | |
|---|---|---|---|---|
| *Mean ± SEM (n = 5); *p<0.001 without NCIS vs. with NCIS; t -Student* | | | | |

### H.2 Coagulation assays in coagulation factor-deficient plasma

### Effect of the rTF, Dade® Actin® reagent and FXa combination

A series of *in vitro* coagulation assays were performed showing the coagulant effect of rTF associated with negatively charged inorganic surfaces [NCIS] (Dade® Actin® FS reagent) and FXa in plasma from coagulation factor-deficient patients (FV, FVII, FVIII, FIX, FXI and FXII).

The procoagulant effect of the combination (rTF + NCIS + FXa) exceeded that obtained when rTF alone was used as a procoagulant agent. Even at low concentrations (1 µg/ml), with which rTF was not able to coagulate the FV-deficient plasma, the combination (rTF + NCIS + FXa) produced a significant procoagulant effect. Likewise, similar synergistic effects were observed when FVII-deficient plasma was used. These results show that the effect mediated by rTF is independent from FVII and is able to induce plasma coagulation even in the absence of FV. The results obtained are shown in Table 22.

**Table 22**

| **Procoagulant effect of rTF associated with (i) negatively charged inorganic surfaces (NCIS), or (ii) with FXa, or (iii) with NCIS and FXa, in coagulation factor-deficient plasma** | | | | | |
|---|---|---|---|---|---|
| **Coagulation time (s)** | | | | | |
| Plasma | Without rTF | With rTF (1 µg/ml) | With rTF (1 µg/ml) + NCIS | With rTF (1 µg/ml) + FXa 80 pg/ml | With rTF (1 µg/ml) + FXa 80 pg/ml + NCIS |
| Normal | 281.1 ± 12.5 | 28.6 ± 0.1 | 16 ± 4.2* | 12.6 ± 3.2* | 8.2 ± 1 |
| FV-D | > 300 | > 300 | 155.2 ± 2.3* | 87.2 ± 1.2* | 62 ± 4* |
| FVII-D | > 300 | 202.7 ± 41.6 | 65 ± 3.4* | 14.8 ± 0.2* | 10.1 ± 2* |
| FVIII-D | > 300 | 32.1 ± 2.6 | 12.1 ± .3.5* | 12.8 ± .0.9* | 9.1 ± 0.4* |
| FIX-D | > 300 | 34.6 ± 2.4 | 14.3 ± 0.2* | 15.6 ± 1.6* | 11.3 ± 0.2* |
| FXI-D | > 300 | 27.3 ± 0.6 | 16.2 ± 1.9* | 14.9 ± 2.7* | 8.1 ± 0.2* |
| FXII-D | > 300 | 32.5 ± 0.5 | 11.5 ± 0.6* | 12.4 ± 2.9* | 8.2 ± 0.4* |

| | | | | | |
|---|---|---|---|---|---|
| *Mean ± SEM (n = 5); * p<0.001 without NCIS vs. with NCIS; t -Student* FV-D: FV-deficient; FVII-D: FVII-deficient; FVIII-D: FVIII-deficient; FIX-D: FIX-deficient; FXI-D: FXI-deficient; FXII-D: FXII-deficient | | | | | |

### II.2 In vivo assays

### I. rTF (alone, or combined with negatively charged inorganic surfaces (NCIS), or combined with low FXa concentrations) is a useful agent for topical treatment of hemorrhaging

*In vivo* assays were performed showing that rTF administered alone or associated with (i) negatively charged inorganic surfaces [NCIS] (Dade® Actin® FS reagent) or rather with (ii) low FXa concentrations, is a useful agent for topical antihemorragic treatment.
The use of rTF as a topical hemostatic agent administered alone or combined with (i) NCIS or with (ii) low FXa concentrations, was evaluated by means of the use of a severe hemorrhage in an animal model by proximal section of rat tails.

The results obtained are shown in Table 23. As can be seen, in said severe hemorrhage model, the hemorrhage spontaneously coagulated in the control animals (PSS Control, treated with physiological saline solution) at 330 seconds; however, topical administration of rTF (alone) produced a significant reduction (143 seconds, p<0.001). When rTF was administered in combination with (i) NCIS and with (ii) low doses of FXa, the procoagulant effect was even greater (77 and 55 seconds, respectively; p<0.001).

**Table 23**

| **Severe hemorrhage model by proximal section of rat tails** | | | |
|---|---|---|---|
| **Coagulation time** | | | |
| | **PSS Control** | | **rTF 10 µg/ml** |
| **animal no. 1** | 306 | **animal no.** 8 | 66 |
| **animal no. 2** | 378 | **animal no. 9** | 132 |
| **animal no. 3** | 252 | **animal no. 10** | 186 |
| **animal no. 4** | 318 | **animal no. 11** | 186 |
| **animal no. 5** | 408 | **animal no. 12** | 126 |
| **animal no. 6** | 426 | **animal no. 13** | 114 |
| **animal no. 7** | 228 | **animal no. 14** | 192 |
| *Mean ± SD* | *330.9 ± 76.2* | | *143.1 ± 47* |

| | **rTF + NCIS** | | **rTF + FXa** |
|---|---|---|---|
| **animal no. 15** | 58 | **animal no. 22** | 35 |
| **animal no. 16** | 111 | **animal no. 23** | 65 |
| **animal no. 17** | 85 | **animal no. 24** | 49 |
| **animal no. 18** | 78 | **animal no. 25** | 47 |
| **animal no. 19** | 49 | **animal no. 26** | 49 |
| **animal no. 20** | 56 | **animal no. 27** | 55 |
| **animal no. 21** | 102 | **animal no. 28** | 86 |
| *Mean* ± *SD* | *77 ± 23.9* | | *55.1 ± 16.3* |

| | | | |
|---|---|---|---|
| *The results are expressed as the time in seconds to reach consolidated coagulation* ◆ *rTF administered topically by contact (1 ml of rTF 10 µg*/*ml) in a well of a 6-well plate.* *◆ rTF + FXa administered topically by contact (1 ml of rTF 10 µg*/*ml + 1 ml of FXa 400 ng*/*ml) in a well of a 6-well plate.* ◆ *rTF + NCIS administered topically by contact (1 ml of rTF 10 µg*/*ml + 1 ml of NCIS) in a well of a 6-well plate.* | | | |

### J. TF (alone, or combined with low FXa concentrations, or combined with negatively charged inorganic surfaces (NCIS)) is a useful agent for antihemorrhagic treatment by applying it directly on the injured blood vessel

*In vivo* assays were performed showing that rTF administered alone or associated with (i) negatively charged inorganic surfaces [NCIS] (Dade® Actin® FS reagent) or with (ii) low FXa concentrations, is a useful agent for topical antihemorrhagic treatment directly applied on the blood vessel.

The use of rTF administered alone or combined with (i) NCIS or with (ii) low FXa concentrations as a topical hemostatic agent was evaluated by means of the use of a fatal hemorrhage in an animal model by puncture in the carotid artery.

The results obtained are shown in Table 24 and were very significant. As can be seen, in the group of control animals, (PSS Control, treated with physiological saline solution), all the animals died from bleeding, whereas in the different treatment groups with rTF alone or combined, no animal died and the section could be successfully sealed and coagulated in all cases. The rTF combination with low doses of FXa was found to be the most effective in terms of the time necessary to achieve the stable coagulation and sealing of the puncture wound.

**Table 24**

| **Very severe (fatal) hemorrhage model by puncture in carotid artery of rats** | | | |
|---|---|---|---|
| **Coagulation time** | | | |
| | **Control SF** | | **rTF** |
| **animal no. 1** | Death | **animal no. 5** | 65 |
| **animal no. 2** | Death | **animal no. 6** | 85 |
| **animal no. 3** | Death | **animal no. 7** | 102 |
| **animal no. 4** | Death | **animal no. 8** | 145 |
| *Mean ± SD* | | | *99.3 ± 34* |

| | **rTF + NCIS** | | **rTF + FXa** |
|---|---|---|---|
| **animal no. 13** | 89 | **animal no. 9** | 55 |
| **animal no. 14** | 65 | **animal no.10** | 49 |
| **animal no. 15** | 81 | **animal no. 11** | 65 |
| **animal no. 16** | 78 | **animal no. 12** | 66 |
| *Mean ± SD* | *78.3 ± 10* | | *58.8 ± 8.2* |

| | | | |
|---|---|---|---|
| *The results are expressed as the time in seconds to reach consolidated coagulation* *◆ rTF administered with pipette (5 ml of rTF 10 µg*/*ml) and dressing on puncture (rTF 10 µg*/*ml).* *◆ rTF + FXa administered with pipette (2.5 ml of rTF 10 µg*/*ml + 2.5 ml of FXa 400 ng*/*ml) and dressing containing the mixture on puncture.* *◆ rTF + NCIS administered with pipette (2.5 ml of rTF 10 µg*/*ml + 2.5 ml of NCIS) and dressing on puncture.* | | | |

## Claims

1. Use of the lipidated Tissue Factor (TF), or a functional fragment thereof, in the manufacture of a medicament for the topical treatment of hemorrhages in a subject.

2. Use according to claim 1, where said subject is a healthy subject or a subject with a hemorrhagic diathesis, where said hemorrhagic diathesis comprises a coagulopathy and/or a platelet disorder.

3. Use according to claim 2, where said coagulopathy is a congenital coagulopathy or an acquired coagulopathy.

4. Use according to claim 3, where said congenital coagulopathy is a coagulopathy based on a deficiency of a coagulation factor chosen from coagulation Factor V, coagulation Factor VII, coagulation Factor VIII, coagulation Factor IX, coagulation Factor X, coagulation Factor XII, coagulation Factor XIII and combinations thereof.

5. Use according to claim 2, where said subject has a congenital or acquired platelet disorder.

6. Use according to claim 5, where said congenital platelet disorder is selected from Glanzamnn's disease, Bernard Soulier syndrome, Bolin-Jamieson's syndrome, Wiskott-Aldrich syndrome, Paris-Trousseau-Jacobsen syndrome, thrombocytopenia of the X chromosome, the Gray platelet syndrome, Sebastian syndrome and Fanconi anemia.

7. Use according to claim 5, where said acquired platelet disorder is selected from a myeloproliferative disorder, such as thrombocytemia, polycytemia, or chronic myelocytic leukemia; myeloid metaplasia; disproteinemias in scurvy, in congenital heart disease and in cirrhosis.

8. Use according to claim 1, where said lipidated Tissue Factor is human lipidated Tissue Factor.

9. A product comprising (i) lipidated Tissue Factor (TF) and (ii) activated coagulation Factor X (FXa).

10. A product comprising, separately, (i) lipidated Tissue Factor (TF) and (ii) activated coagulation Factor X (FXa).

11. A product comprising, separately, (i) lipidated Tissue Factor (TF) and (ii) activated coagulation Factor X (FXa) as a combination for their simultaneous or successive administration to a subject.

12. A product comprising (i) lipidated Tissue Factor (TF) and (ii) a negatively charged inorganic surface (NCIS).

13. A product comprising, separately, (i) lipidated Tissue Factor (TF) and (ii) a negatively charged inorganic surface (NCIS).

14. A product comprising, separately, (i) lipidated Tissue Factor (TF) and (ii) a negatively charged inorganic surface (NCIS) as a combination for their simultaneous or successive administration to a subject.

15. A product comprising (i) lipidated Tissue Factor (TF), (ii) activated coagulation Factor X (FXa) and (iii) a negatively charged inorganic surface (NCIS).

16. A product comprising, separately, (i) lipidated Tissue Factor (TF), (ii) activated coagulation Factor X (FXa) and (iii) a negatively charged inorganic surface (NCIS).

17. A product comprising, separately, (i) lipidated Tissue Factor (TF), (ii) activated coagulation Factor X (FXa) and (iii) a negatively charged inorganic surface (NCIS) as a combination for their simultaneous or successive administration to a subject.

18. A product according to any of claims 9 to 17, where said lipidated Tissue Factor is human lipidated Tissue Factor.

19. A product according to any of claims 9 to 18, as a medicament.

20. Use of a product according to any of claims 9 to 18 in the manufacture of a medicament for the treatment of hemorrhages in a subject.

21. Use of a product according to any of claims 9 to 18 in the manufacture of a medicament for the topical treatment of hemorrhages in a subject.

22. A complex (TF::FXa) comprising lipidated Tissue Factor (TF) and activated coagulation Factor X (FXa).

23. A complex according to claim 22, as a medicament.

24. Use of a complex according to claim 22 in the manufacture of a medicament for the treatment of hemorrhages in a subject.

25. A complex (TF::NCIS) comprising lipidated Tissue Factor (TF) and a negatively charged inorganic surface (NCIS).

26. A complex according to claim 25, as a medicament.

27. Use of a complex according to claim 25 in the manufacture of a medicament for the treatment of hemorrhages in a subject.

28. Use of a complex according to claim 25 in the manufacture of a medicament for the topical treatment of hemorrhages in a subject.

29. A complex (TF::FXa::NCIS) comprising lipidated Tissue Factor (TF), activated coagulation Factor X (FXa) and a negatively charged inorganic surface (NCIS).

30. A complex according to claim 29, as a medicament.

31. Use of a complex according to claim 29 in the manufacture of a medicament for the treatment of hemorrhages in a subject.

32. Use of a complex according to claim 29 in the manufacture of a medicament for the topical treatment of hemorrhages in a subject.

33. A pharmaceutical composition comprising a lipidated Tissue Factor (TF), together with a pharmaceutically acceptable vehicle.

34. A pharmaceutical composition for the topical administration of a lipidated Tissue Factor (TF), comprising lipidated TF and a pharmaceutically acceptable vehicle suitable for the topical administration of said lipidated TF.

35. A pharmaceutical composition comprising:
a) a product according to claim 9, together with a pharmaceutically acceptable vehicle; or
b) separately, (i) lipidated TF together with a pharmaceutically acceptable vehicle, and (ii) FXa together with a pharmaceutically acceptable vehicle; or
c) a product according to claim 12, together with a pharmaceutically acceptable vehicle; or
d) separately, (i) lipidated TF together with a pharmaceutically acceptable vehicle, and (ii) a negatively charged inorganic surface (NCIS) together with a pharmaceutically acceptable vehicle; or
e) a product according to claim 15, together with a pharmaceutically acceptable vehicle; or
f) separately, (i) lipidated TF together with a pharmaceutically acceptable vehicle, (ii) FXa together with a pharmaceutically acceptable vehicle, and (iii) a negatively charged inorganic surface (NCIS) together with a pharmaceutically acceptable vehicle; or
g) a TF::FXa complex, according to claim 22, together with a pharmaceutically acceptable vehicle; or
h) a TF::NCIS complex, according to claim 25, together with a pharmaceutically acceptable vehicle; or
i) a TF::FXa::NCIS complex, according to claim 29, together with a pharmaceutically acceptable vehicle.

36. A pharmaceutical composition according to claim 35, in a pharmaceutical administration form for its topical administration.

37. A product comprising a pharmaceutical composition according to any of claims 33 to 36 and a support.

38. A product comprising a pharmaceutical composition comprising:
(i) a support, (ii) a product according to claim 9, together with a pharmaceutically acceptable vehicle, (iii) a product according to claim 12, together with a pharmaceutically acceptable vehicle and (iv) a product according to claim 15, together with a pharmaceutically acceptable vehicle; or
a TF::FXa complex according to claim 22, together with a pharmaceutically acceptable vehicle, or
a TF::NCIS complex according to claim 25, together with a pharmaceutically acceptable vehicle, or
a TF::FXa::NCIS complex according to claim 29, together with a pharmaceutically acceptable vehicle.

39. Use of the non-lipidated Tissue Factor (TF), or a functional fragment thereof, for the manufacture of a medicament for the treatment of hemorrhages in a coagulation Factor VII-deficient subject.

40. Use according to claim 39 for the manufacture of a medicament for parenteral administration of non lipidated TF or a fragment thereof.
